# EUROPEAN PATENT SPECIFICATION

(11) **EP 1 796 622 B1**
(45) Date of publication and mention of the grant of the patent: **23.01.2008**
(21) Application number: 05761918.1
(22) Date of filing: 13.06.2005
(51) Int. Cl.: A61K 8/18

(54) **COSMETIC COMPOSITIONS CONTAINING A TRIAZINE DERIVATIVE, AN AMIDE-BASED OIL AND A EUTECTIC MIXTURE OF N-BUTYL-PHTHALIMIDE/ISOPROPYLPHTHALIMIDE**
TRIAZINDERIVAT, AMID-BASIERTES ÖL UND EIN EUTEKTISCHES GEMISCH AUS N-BUTYL-PHTHALIMID/ISOPROPYLPHTHALIMID ENTHALTENDE KOSMETISCHE ZUSAMMENSETZUNGEN
COMPOSITIONS COSMETIQUES CONTENANT UN DERIVE DE TRIAZINE, UNE HUILE A BASE D'AMIDES ET UN MELANGE EUTECTIQUE DE N-BUTYL-PHTHALIMIDE/ISOPROPYLPHTHALIMIDE

(30) Priority: 02.07.2004 FR 0451424; 20.07.2004 US 589015 P
(43) Date of publication of application: 20.06.2007
(73) Proprietor: L'ORÉAL, 75008 Paris (FR)
(72) Inventor: CANDAU, Didier, F-91570 Bievres (FR); FIANDINO, Cécile, F-75013 Paris (FR)
(74) Representative: Miszputen, Laurent
(86) International application number: PCT/EP2005/007888
(87) International publication number: WO 2006/003028

(56) References cited:
- EP-A- 1 082 954
- EP-A- 1 269 980
- EP-A- 1 430 881
- WO-A-03/039447

## Description

The present invention relates to a photoprotective composition comprising at least one 1,3,5-triazine derivative, at least one amide-based oil and a eutectic mixture of n-butylphthalimide/isopropylphthalimide.

The present invention also relates to the various cosmetic uses of the said compositions, especially for protecting the skin and/or the lips and/or the integuments against ultraviolet radiation, in particular solar radiation.

It is known that light radiation with wavelengths of between 280 nm and 400 nm permits tanning of the human epidermis, and that rays with wavelengths of between 280 nm and 320 nm, known as UV-B rays, cause skin burns and erythema which may be harmful to the development of a natural tan; this UV-B radiation should thus be screened out.

It is also known that UV-A rays, with wavelengths of between 320 nm and 400 nm, which cause tanning of the skin, are liable to induce an adverse change in the latter, especially in the case of sensitive skin or of skin which is continually exposed to solar radiation. UV-A rays cause in particular a loss of elasticity of the skin and the appearance of wrinkles, leading to premature skin aging. They promote triggering of the erythemal reaction or amplify this reaction in certain individuals and may even be the cause of phototoxic or photoallergic reactions. It is thus desirable also to screen out UV-A radiation.

Many cosmetic compositions intended for the photoprotection (UV-A and/or UV-B) of the skin have been proposed to date.

These antisun compositions are quite often in the form of an emulsion of oil-in-water type (that is to say a cosmetically acceptable support consisting of a continuous aqueous dispersing phase and a discontinuous oily dispersed phase) which contains, in various concentrations, one or more conventional lipophilic and/or hydrophilic organic screening agents which are capable of selectively absorbing harmful UV radiation, these screening agents (and their amounts) being selected as a function of the desired sun protection factor, the sun protection factor (SPF) being expressed mathematically by the ratio of the UV radiation dose necessary to reach the erythema-forming threshold with the UV screening agent to the UV radiation dose necessary to reach the erythema-forming threshold without UV screening agent.

1,3,5-Triazine derivatives are particularly desired in antisun cosmetics due to the fact that they are highly active in the UV-B range, and even in the UV-A range for some of these compounds depending on the nature of the substituents involved. Furthermore, they are photostable, i.e. they show little or no chemical degradation under the action of UV radiation. They are especially described in patent applications US 4 367 390, EP 863 145, EP 517 104, EP 570 838, EP 796 851, EP 775 698, EP 878 469 and EP 933 376, and the following are known in particular:
- 2,4,6-tris[p-(2'-ethylhexyl-1'-oxycarbonyl)anilino]-1,3,5-triazine or "Ethylhexyl Triazone" (INCI name), sold under the trade name "Uvinul T 150" by the company BASF,
- 2-[(p-(tert-butylamido)anilino]-4,6-bis-[(p-(2'-ethylhexyl-1'-oxycarbonyl)anilino]-1,3,5-triazine or "Diethylhexyl Butamido Triazone" (INCI name), sold under the trade name "Uvasorb HEB" by Sigma 3V,
- 2,4-bis{[4-2-ethylhexyloxy)]-2-hydroxy]phenyl}-6-(4-methoxyphenyl)-1,3,5-triazine or "Bis-Ethylhexyloxyphenol Methoxyphenyl Triazine" (INCI name), sold under the trade name "Tinosorb S" by Ciba Specialty Chemicals.

It has been proposed in the prior art to use 1,3,5-triazine derivatives in oils such as esters and more particularly C₁₂-C₁₅ alkyl benzoates ("Finsolv TN" from Finetex), or triglycerides and especially C₈-C₁₂ fatty acid triglycerides ("Miglyol 812" from Hüls), or alternatively oxyethylenated or oxypropylenated fatty monoalcohols or polyols ("Cetiol HE" from Henkel or "Witconol AM" from Witco). The use of these oils presents two drawbacks:
- either the appearance over time of crystallization in the formulations, which is detrimental to the cosmetic qualities, the stability and the efficacy of antisun products;
- or the limitation of the concentration of screening agents in the formulations, which does not make it possible to obtain products that are sufficiently effective.

The technical problem underlying the present invention is thus that of improving the photoprotective efficacy, the cosmetic properties and the stability of compositions containing such 1,3,5-triazine derivatives.

With this aim, it has already been proposed in patent EP 0 748 623 to add to 2,4,6-tris[p-(2'-ethylhexyl-1'-oxycarbonyl)anilino]-1,3,5-triazine or "Ethylhexyl Triazone" N,N-disubstituted amide-based compounds without emulsifying properties, for instance N,N-diethyl-3-methylbenzamide or ethyl N-butyl-N-acetylaminopropionate.

It has also been proposed in patent EP 1 269 980 to add to UV-screening agents of triazine type N-acyl esters of amino acids, for instance isopropyl N-lauroylsarcosinate.

The results obtained with these amide-based oils are still not entirely satisfactory.

It has also been proposed in patent EP 1 430 881 to add a eutectic mixture of n-butylphthalimide/isopropylphthalimide to UV-screening agents of triazine type.

The results obtained with this eutectic mixture are still not entirely satisfactory.

Surprisingly and unexpectedly, the Applicant has discovered that the technical problem mentioned above can be solved by using a combination of an oil containing in its structure at least one amide unit and a eutectic mixture of n-butylphthalimide/isopropylphthalimide in a composition containing at least one triazine derivative.

This combination makes it possible to obtain stable antisun compositions containing 1,3,5-triazine derivatives, which have a sun protection factor higher than those of the prior art compositions containing 1,3,5-triazine derivatives. These compositions also have improved cosmetic qualities. They especially allow good moisturization of the skin, i.e. no dryness of the skin and no greasy feel are observed.

One subject of the present invention is thus a photoprotective cosmetic or dermatological composition containing
a) at least one 1,3,5-triazine derivative and
b) at least one oil containing in its structure at least one amide unit and
c) at least one eutectic mixture of n-butylphthalimide and of isopropylphthalimide.

Another subject of the invention consists of the use of such a composition for the manufacture of cosmetic or dermatological compositions intended in particular for protecting keratin materials against solar radiation.

A subject of the invention is also the use of a combination of (i) at least one oil containing in its structure at least one amide unit and (ii) a eutectic mixture of n-butylphthalimide and of isopropylphthalimide in a photoprotective cosmetic or dermatological composition containing at least one 1,3,5-triazine derivative, in order to improve the sun protection factor and/or the cosmetic qualities and/or the stability of this composition.

Other subjects of the invention will become apparent on reading the description and the examples that follow.

The expression "photoprotective composition" means any composition containing at least one organic compound and/or at least one mineral compound capable of screening out UV-A and/or UV-B radiation via a phenomenon of absorption, reflection or scattering.

The expression "oil containing in its structure at least one amide unit" will be understood throughout the text of the description to mean any compound comprising in its chemical structure at least one amide group (or function) of the type: 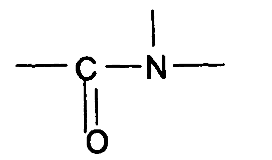 and simultaneously having the following characteristics:
a) liquid at 25°C,
b) insoluble or immiscible in water at 25°C,
c) no emulsifying properties.

Preferably, the combination of eutectic mixture of n-butylphthalimide and of isopropylphthalimide/amide-based oil is present in the composition in a sufficient amount to make it possible by itself to dissolve (without it being necessary to use another solvent) the total amount of triazine screening agent present in the composition.

The oil(s) having in their structure at least one amide unit in accordance with the invention is (are) preferably chosen from the compounds of formula (1a) or (1b) below: 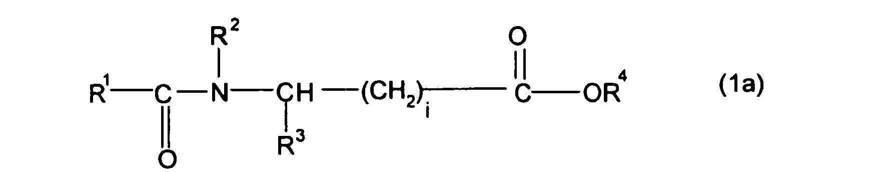 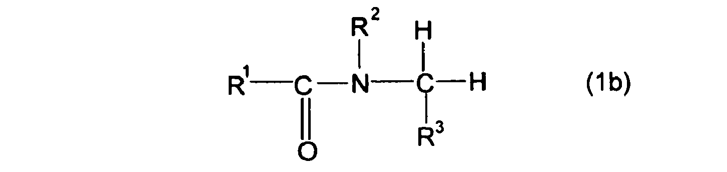 in which:
- the radical R¹ represents an optionally functionalized, aliphatic, cycloaliphatic or cyclic, saturated or unsaturated monovalent hydrocarbon-based radical containing from 1 to 30 carbon atoms and preferably from 1 to 22 carbon atoms, limits inclusive;
- the radicals R², R³ and R⁴, which may be identical or different, represent optionally functionalized, aliphatic, cycloaliphatic or cyclic, saturated or unsaturated monovalent hydrocarbon-based radicals containing from 1 to 30 carbon atoms and preferably from 1 to 22 carbon atoms, limits inclusive;
- i is an integer from 0 to 2;

Examples of saturated aliphatic hydrocarbon-based radicals that may especially be mentioned include linear or branched, substituted or unsubstituted C₁-C₃₀ and preferably C₁-C₂₂ alkyl radicals, and in particular methyl, ethyl, n-propyl, isopropyl, n-butyl, isobutyl, tert-butyl, pentyl, n-amyl, isoamyl, neopentyl, n-hexyl, n-heptyl, n-octyl, 2-ethylhexyl, tert-octyl, decyl, lauryl and octadecyl radicals.

Examples of saturated cyclic hydrocarbon-based radicals that may especially be mentioned include cyclopentyl and cyclohexyl radicals, which are optionally substituted, in particular with alkyl radicals.Examples of unsaturated aliphatic hydrocarbon-based radicals that may especially be mentioned include linear or branched, substituted or unsubstituted, C₂-C₃₀ and preferably C₂-C₂₂ alkenyl or alkynyl radicals, and in particular vinyl, allyl, oleyl and linoleyl radicals.

Examples of unsaturated cyclic hydrocarbon-based radicals that may especially be mentioned include aryl radicals such as phenyl and naphthyl, which are optionally substituted, in particular with alkyls, for instance a tolyl radical, and examples of unsaturated cycloaliphatic radicals that may be mentioned more particularly include benzyl and phenylethyl radicals.

The term "functionalized radicals" more particularly means radicals comprising in their chemical structure, either in the main chain or on a secondary chain unit, one or more functional groups especially such as esters, ethers, alcohols, amines, amides and ketones, but preferably esters.

The preferred amide-based oils of formula **(1a) or (1b)** are chosen from those in which:
R¹ represents a linear or branched C₁-C₂₂ alkyl radical; a linear or branched C₂-C₂₂ alkenyl radical; an aryl radical;
R² represents a hydrogen atom or a linear or branched C₁-C₆ alkyl group;
R³ represents a hydrogen atom or a linear or branched C₁-C₆ alkyl group;
R⁴ represents a linear or branched C₁-C₁₀ alkyl radical or a linear or branched C₂-C₁₀ alkenyl radical or a sterol residue.
In formula **(1a) or (1b)** presented above, the group R¹(CO)- is an acyl group of an acid preferably chosen from the group formed by acetic acid, toluic acid, capric acid, lauric acid, myristic acid, palmitic acid, stearic acid, behenic acid, linoleic acid, linolenic acid, oleic acid, isostearic acid, 2-ethylhexanoic acid, coconut oil fatty acids and palm kernel oil fatty acids. These acids may also contain a hydroxyl group.

In formula (1a), the portion -N (R²) CH (R³) (CH₂)q(CO)- of the amino acid ester is preferably chosen from those corresponding to the following amino acids: glycine, alanine, valine, leucine, isoleucine, serine, threonine, proline, hydroxyproline, β-alanine, N-butyl-β-alanine, aminobutyric acid, aminocaproic acid, sarcosine or N-methyl-β-alanine .

In formula (1a), " the portion of the amino acid esters corresponding to the group OR⁴ may be obtained from alcohols chosen from the group formed by methanol, ethanol, propanol, isopropanol, butanol, tert-butanol, isobutanol, 3-methyl-1-butanol, 2-methyl-1-butanol, pentanol, hexanol, cyclohexanol, octanol, 2-ethylhexanol, decanol, lauryl alcohol, myristyl alcohol, cetyl alcohol, cetostearyl alcohol, stearyl alcohol, oleyl alcohol, behenyl alcohol, jojoba alcohol, 2-hexadecyl alcohol, 2-octyldodecanol and isostearyl alcohol.

The oils containing in their structure at least one amide function of formula (1a) or (1b) in accordance with the invention are known per se. Some of them are especially described with their methods of preparation in patent applications EP 1 044 676 and EP 0 928 608 from the company Ajinomoto Co. Others are known in cosmetics, for instance insect repellents such as ethyl N-acetyl-N-butylaminopropionate or N,N-diethyltoluamide.

Among the compounds of formula **(1a) or (1b)** that are particularly preferred, mention may be made of:
ethyl N-acetyl-N-butylaminopropionate, having the following formula: 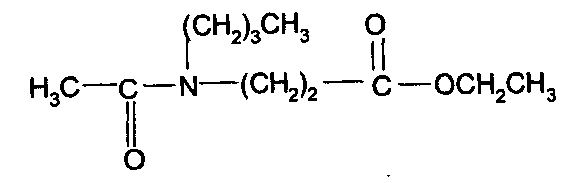 such as the product sold under the trade name Repellent R3535 by the company Merck;
isopropyl N-lauroylsarcosinate of formula: 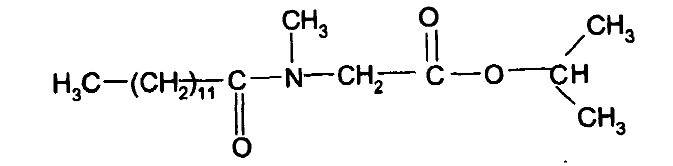 such as the product sold under the name Eldew SL-205 by the company Ajimoto;
N,N-diethyltoluamide of formula: 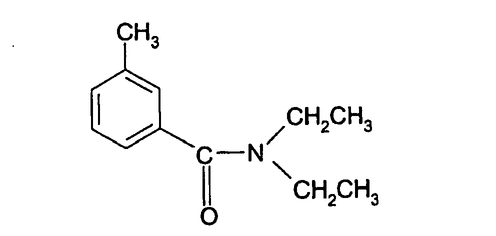 such as the product sold under the trade name Deet by the company Showa Denko.

The oil(s) containing in their structure at least one amide function as defined above is (are) present in the compositions according to the invention in concentrations preferably ranging from 0.1% to 40% by weight and more preferably from 1% to 20% by weight relative to the total weight of the composition.

The eutectic mixture in accordance with the invention preferably consists of:
(a) 60% to 75% by weight of n-butylphthalimide of structure (2) below: 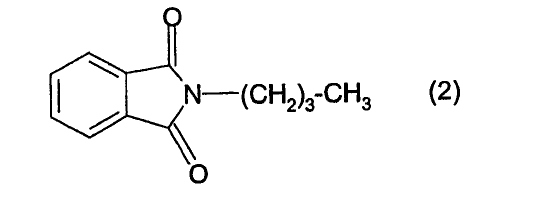
(b) 25% to 40% by weight of isopropylphthalimide in accordance with the invention, corresponding to structure (3) below:

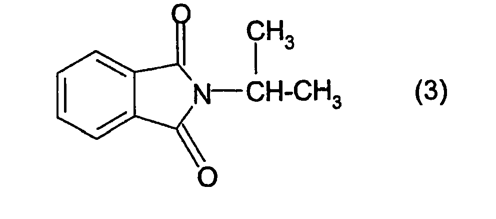

The n-butylphthalimide/isopropylphthalimide eutectic mixtures in accordance with the invention are known and are described and synthesized in patent US 6 306 373 (which forms an integral part of the content of the description).

Among the eutectic mixtures that may be used, mention may be made of the following mixtures:
n-butylphthalimide/isopropylphthalimide (60/40% by weight)
n-butylphthalimide/isopropylphthalimide (62/38% by weight)
n-butylphthalimide/isopropylphthalimide (65/35% by weight)
n-butylphthalimide/isopropylphthalimide (70/30% by weight)
n-butylphthalimide/isopropylphthalimide (75/25% by weight).

The n-butylphthalimide/isopropylphthalimide eutectic mixture sold under the name Pelemol BIP by the company Phoenix Chemicals will be used, for example.

The eutectic mixture in accordance with the invention will preferably be used at concentrations ranging from 0.1% to 40% by weight and more preferably from 1% to 30% by weight relative to the total weight of the composition.

The 1,3,5-triazine derivative corresponds to formula (I) below: 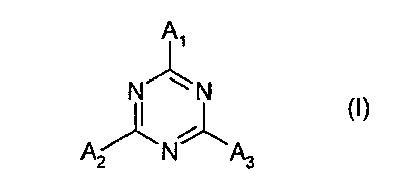 in which the radicals A₁, A₂ and A₃, which may be identical or different, are chosen from the groups of formulae (II) to (IX) below: 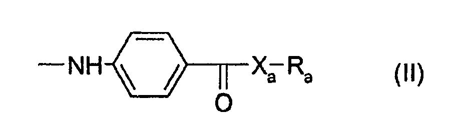 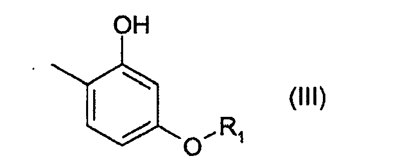 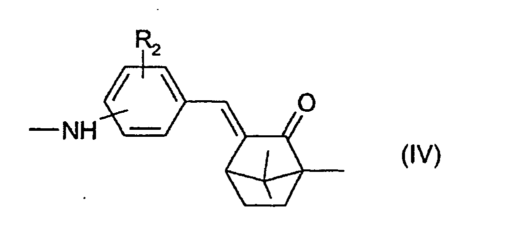 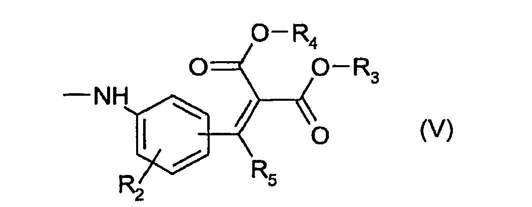 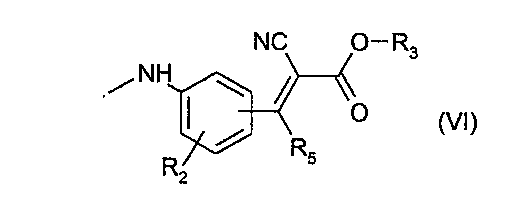 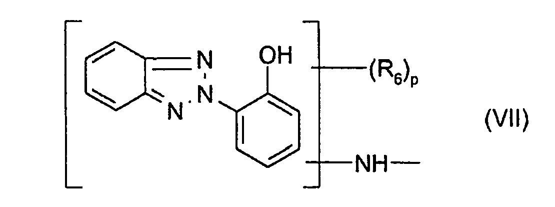 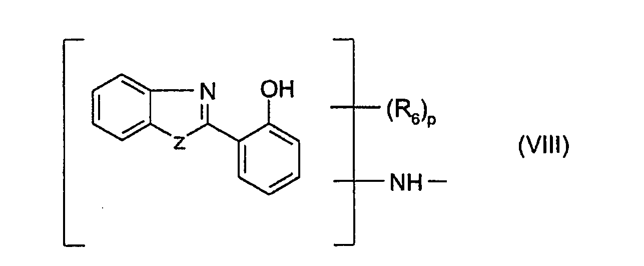 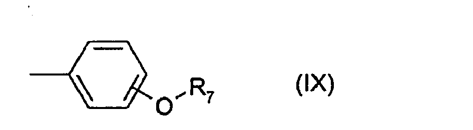 in which:
- Xₐ (each of the groups Xₐ may be identical or different) represents oxygen or -NH-;
- Rₐ (each of the groups Rₐ may be identical or different) is chosen from hydrogen; an alkali metal; an ammonium radical optionally substituted with one or more linear or branched C₁-C₁₈ alkyl or linear or branched C₁-C₁₈ hydroxyalkyl radicals; a linear or branched C₁-C₁₈ and preferably C₆-C₁₂ alkyl radical; a C₅-C₁₂ cycloalkyl radical optionally substituted with one or more C₁-C₄ alkyl radicals; a polyoxyethylenated radical comprising from 1 to 6 ethylene oxide units, the terminal OH group of which is methylated; a radical of formula (X), (XI) or (XII) below: 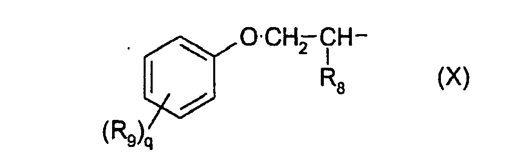 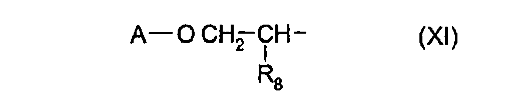 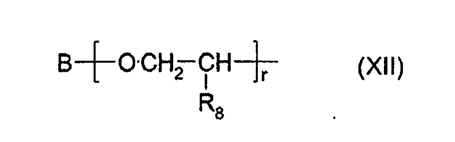
in which:
- R₈ is hydrogen or a methyl radical;
- R₉ is a C₁-C₉ alkyl radical;
- q is an integer equal to 0; 1; 2; 3;
- r is an integer equal to 1; 2; 3; 4; 5; 6; 7; 8; 9; 10;
- A is a C₄-C₈ alkyl radical or a C₅-C₈ cycloalkyl radical;
- B is chosen from: a linear or branched C₁-C₈ alkyl radical; a C₅-C₈ cycloalkyl radical; an aryl radical optionally substituted with one or more C₁-C₄ alkyl radicals;
- R₁ denotes a C₃-C₁₈ alkyl radical; a C₂-C₁₈ alkenyl radical; a residue of formula -CH₂-CH(OH)-CH₂-OT₁ in which T₁ is a hydrogen atom or a C₁-C₈ alkyl radical; a residue of formula (XIII) below: 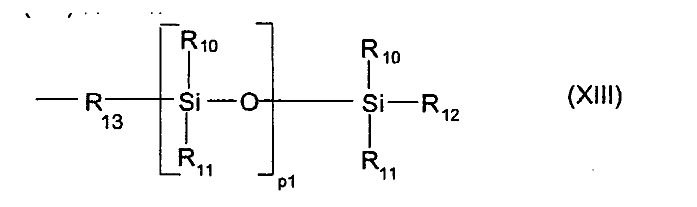
in which:
- R₁₃ denotes a covalent bond; a linear or branched C₁-C₄ alkyl radical or a radical of formula -Cₘ₁H₂ₘ₁-O- in which m₁ is an integer equal to 1; 2; 3; 4;
- p₁ is an integer equal to 0; 1; 2; 3; 4; 5;
- the radicals R₁₀, R₁₁ and R₁₂, which may be identical or different, denote a C₁-C₁₈ alkyl radical; a C₁-C₁₈ alkoxy radical or a radical of formula: 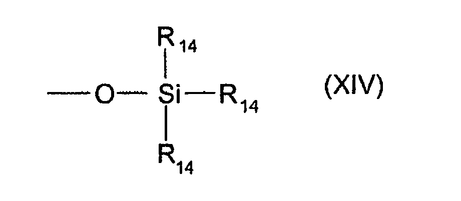
in which R₁₄ is a C₁-C₅ alkyl radical;
- R₂ denotes a hydrogen atom, a linear or branched C₁-C₄ alkyl radical or a C₁-C₄ alkoxy radical;
- R₃ and R₄, which may be identical or different, denote a linear or branched C₁-C₂₀ alkyl radical;
- R₅ represents a hydrogen atom or a phenyl radical optionally substituted with a halogen or with a C₁-C₄ alkyl radical or with a C₁-C₄ alkoxy radical;
- R₆ is a linear or branched C₁-C₈ alkyl radical or a C₁-C₃ alkoxy radical, it being understood that, in the latter case, two adjacent radicals R₆ on the same aromatic nucleus can together form an alkylidenedioxy group in which the alkylidene group contains 1 or 2 carbon atoms, OH, NHCOCH₃ or NH₂;
- R₇ denotes a hydrogen atom, a C₁-C₁₀ alkyl radical, a radical of formula: -(CH₂CHR₅-O)ₙ₁R₈ in which n₁ is a number from 1 to 16, or a radical of structure -CH₂-CH-(OH)-CH₂OT₁ with R₈ and T₁ having the same meaning as indicated above;
- Z represents oxygen, sulfur, -NH- or -NR₃- with R₃ representing a linear or branched C₁-C₂₀ alkyl radical;
- p is 0, 1, 2 or 3;
A₁ can also be a halogen, a radical -N(R₃)₂, the two radicals R₃ together possibly forming a ring of 4 or 5 carbon atoms, or a group -OR₃, R₃ having the same definition as above.

A first family of 1,3,5-triazine derivatives that is more particularly preferred, and that is described especially in document EP-A-0 517 104, is that of the 1,3,5-triazines corresponding to formula (I) in which A₁, A₂ and A₃ are of formula (II) and have all of the following characteristics:
- one of the groups Xₐ-Rₐ represents a radical-NH-Rₐ with Rₐ chosen from: a C₅-C₁₂ cycloalkyl radical optionally substituted with one or more C₁-C₄ alkyl radicals; a radical of formula (X), (XI) or (XII) above in which:
- B is a C₁-C₄ alkyl radical;
- R₉ is a methyl radical;
- the other two groups Xₐ-Rₐ represent a radical -O-Rₐ with Rₐ, which may be identical or different, chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a radical of formula (X), (XI) or (XII) above in which:
- B is a C₁-C₄ alkyl radical;
- R₉ is a methyl radical.

A second family of 1,3,5-triazine derivatives that is more particularly preferred, and that is described especially in document EP-A-0 570 838, is that of the 1,3,5-triazines corresponding to formula (I) in which A₁, A₂ and A₃ are of formula (II) and have all of the following characteristics:
- one or two groups Xₐ-Rₐ represent a radical -NH-Rₐ, with Rₐ chosen from: a linear or branched C₁-C₁₈ alkyl radical; a C₅-C₁₂ cycloalkyl radical optionally substituted with one or more C₁-C₄ alkyl radicals; a radical of formula (X), (XI) or (XII) above in which B is a C₁-C₄ alkyl radical and R₉ is a methyl radical;
- the other or the other two group(s) Xₐ-Rₐ being a radical -O-Rₐ with Rₐ, which may be identical or different, chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a radical of formula (X), (XI) or (XII) above in which B is a C₁-C₄ alkyl radical and R₉ is a methyl radical.

A 1,3,5-triazine of this second family that is particularly preferred is 2-[(p-(tert-butylamido)-anilino]-4,6-bis[(p-(2'-ethylhexyl-1'-oxycarbonyl)-anilino]-1,3,5-triazine or "Diethylhexyl Butamido Triazone" sold under the trade name "Uvasorb HEB" by Sigma 3V and corresponding to the following formula: 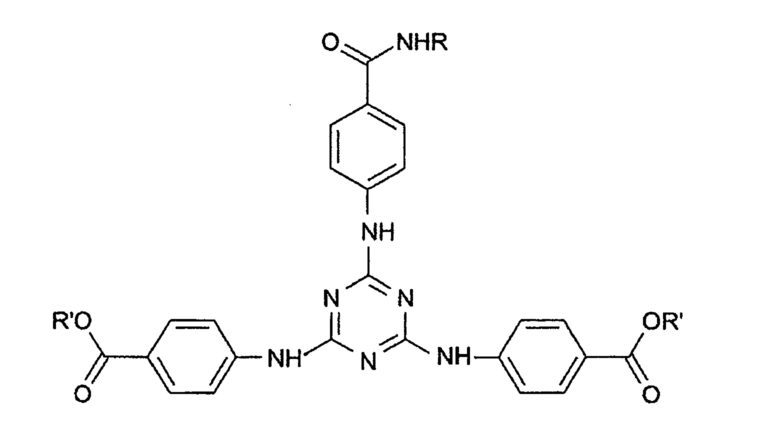 in which R' denotes a 2-ethylhexyl radical and R denotes a tert-butyl radical.

A third preferred family of compounds that may be used in the context of the present invention, and which is described especially in document US 4 724 137, is that of the 1,3,5-triazines corresponding to formula (I) in which A₁, A₂ and A₃ are of formula (II) and have all of the following characteristics:
- Xₐ are identical and represent oxygen;
- Rₐ, which may be identical or different, represent a C₆-C₁₂ alkyl radical or a polyoxyethylenated radical comprising from 1 to 6 ethylene oxide units, the terminal OH group of which is methylated.

A 1,3,5-triazine of this third family that is particularly preferred is 2,4,6-tris[p(2'-ethylhexyl-1'-oxycarbonyl)anilino]-1,3,5-triazine or "Ethylhexyl Triazone" sold especially under the trade name "Uvinul T 150" by the company BASF and corresponds to the following formula: 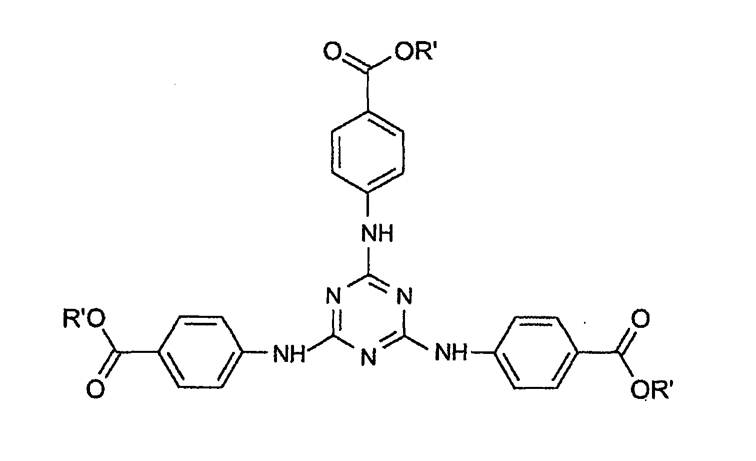 in which R' denotes a 2-ethylhexyl radical.

A fourth preferred family of compounds that may be used in the context of the present invention, and which is described especially in patent application EP-A-0 775 698, is that of the 1,3,5-triazines corresponding to formula (I) in which A₁ and A₂ are of formula (III) and A₃ is of formula (IX) and have all of the following characteristics: R₁, which may be identical or different, denote a C₃-C₁₈ alkyl radical; a C₂-C₁₈ alkenyl radical or a residue of formula -CH₂-CH(OH)-CH₂-OT₁ in which T₁ is a hydrogen atom or a C₁-C₈ alkyl radical; R₇ denotes a hydrogen atom or a C₁-C₁₀ alkyl radical.

A 1,3,5-triazine of this fourth family that is particularly preferred is 2,4-bis{[4-2-ethylhexyloxy)]-2-hydroxy]phenyl}-6-(4-methoxyphenyl)-1,3,5-triazine or "Anisotriazine" sold under the trade name "Tinosorb S" by Ciba Specialty Chemicals, and corresponds to the following formula: 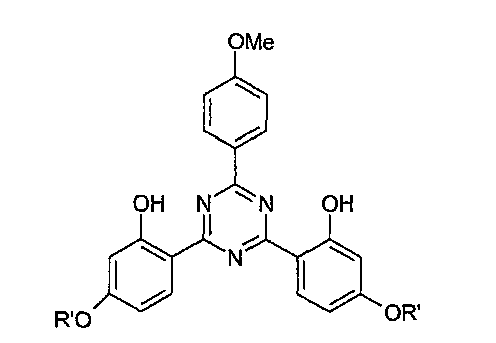 in which R' denotes a 2-ethylhexyl radical.

A fifth preferred family of compounds that may be used in the context of the present invention, and which is described especially in patent applications EP 507 691, EP 507 692, EP 790 243 and EP 944 624, and the technical content of which is incorporated in its entirety in the present description, is that of the 1,3,5-triazines corresponding to formula (I) in which A₁, A₂ and A₃ are of formulae (VII) to (XI) mentioned above.

As examples of these compounds of formula that may be used, mention may be made of:
2,4,6-tris(diisobutyl 4'-aminobenzalmalonate)-s-triazine,
2,4,6-tris(bis(2-ethylhexyl) 4'-aminobenzalmalonate)-s-triazine,
2,4,6-tris(bis(2-ethylhexyl) 4'-aminobenzalmalonate)-6-chloro-s-triazine,
2,4,6-tris(bis(2-ethylhexyl) 4'-aminobenzalmalonate)-6-(2-ethylhexyl 4'-aminobenzoate)-s-triazine,
2,4,6-tris(diisobutyl 4'-aminobenzalmalonate)-6-butoxy-s-triazine,
2,4,6-tris(diisobutyl 4'-aminobenzalmalonate)-6-(2-ethylhexylamino)-s-triazine,
2,4-bis(4'-aminobenzylidenecamphor)-6-(2-ethyl-hexylamino)-s-triazine,
2,4-bis(4'-aminobenzylidenecamphor)-6-(diisobutyl 4'-aminobenzalmalonate)-s-triazine,
2,4,6-tris(diethyl 4'-aminobenzalmalonate)-s-triazine,
2,4,6-tris(diisopropyl 4'-aminobenzalmalonate)-s-triazine,
2,4,6-tris(dimethyl 4'-aminobenzalmalonate)-s-triazine, 2,4,6-tris(ethyl α-cyano-4-aminocinnamate)-s-triazine,
2,4,6-tris[(3'-benzotriazol-2-yl-2'-hydroxy-5'-methyl)phenylamino]-s-triazine,
2,4,6-tris[(3'-benzotriazol-2-yl-2'-hydroxy-5'-tert-octyl)phenylamino]-s-triazine.

A 1,3,5-triazine of this fifth family that is particularly preferred is 2,4,6-tris(diisobutyl 4'-aminobenzalmalonate)-s-triazine, which corresponds to the following formula:

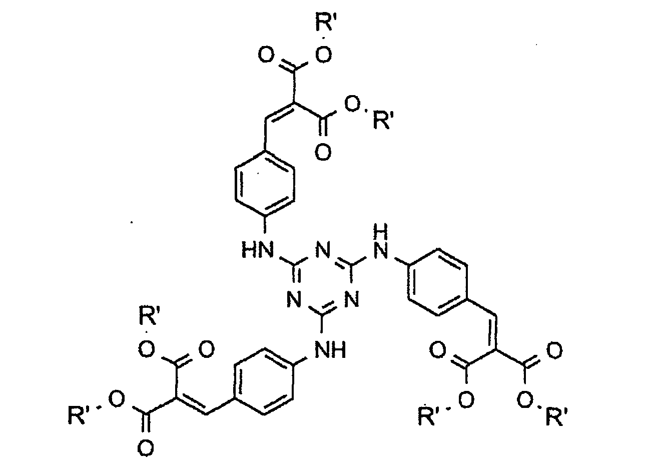

The compositions according to the present invention preferably comprise, in a physiologically acceptable medium, from 0.05% to 15% and preferably from 0.1% to 10% of 1,3,5-triazine derivatives by weight relative to the total weight of the said composition.

The said composition according to the present invention is preferably a cosmetic composition containing, besides the 1,3,5-triazine derivative as organic screening agent, at least one other additional organic screening agent and/or at least one other additional mineral screening agent, which is water-soluble, liposoluble or insoluble in the cosmetic solvents commonly used.

The compositions in accordance with the invention may also comprise other additional UVA-active and/or UVB-active organic or mineral photoprotective agents that are water-soluble or liposoluble or insoluble in the cosmetic solvents commonly used.

The additional organic photoprotective agents are chosen especially from anthranilates; cinnamic derivatives; salicylic derivatives; camphor derivatives; benzophenone derivatives; β,β-diphenylacrylate derivatives; benzotriazole derivatives; benzalmalonate derivatives; benzimidazole derivatives; imidazolines; bis-benzazolyl derivatives as described in patents EP 669 323 and US 2 463 264; p-aminobenzoic acid (PABA) derivatives; methylenebis(hydroxyphenylbenzotriazole) derivatives as described in patent applications US 5 237 071, US 5 166 355, GB 2 303 549, DE 197 26 184 and EP 893 119; benzoxazole derivatives as described in patent applications EP 0 832 642, EP 1 027 883, EP 1 300 137 and DE 101 62 844; screening polymers and screening silicones such as those described especially in patent application WO 93/04665; dimers derived from α-alkylstyrene, such as those described in patent application DE 198 55 649; 4,4-diarylbutadienes such as those described in patent applications EP 0 967 200, DE 197 46 654, DE 197 55 649, EP-A-1 008 586, EP 1 133 980 and EP 133 981, and mixtures thereof.

As examples of additional organic screening agents, mention may be made of those denoted hereinbelow under their INCI name:
para-Aminobenzoic acid derivatives:
   PABA,
   Ethyl PABA,
   Ethyl dihydroxypropyl PABA,
   Ethylhexyl dimethyl PABA sold in particular under the name "Escalol 507" by ISP,
   Glyceryl PABA,
   PEG-25 PABA sold under the name "Uvinul P25" by BASF.
Salicylic derivatives:
   Homosalate sold under the name "Eusolex HMS" by Rona/EM Industries,
   Ethylhexyl salicylate sold under the name "Neo Heliopan OS" by Haarmann and Reimer,
   Dipropylene glycol salicylate sold under the name "Dipsal" by Scher,
   TEA salicylate sold under the name "Neo Heliopan TS" by Haarmann and Reimer.
Cinnamic derivatives:
   Ethylhexyl methoxycinnamate sold in particular under the trade name "Parsol MCX" by Hoffmann LaRoche, Isopropyl methoxycinnamate,
   Isoamyl methoxycinnamate sold under the trade name "Neo Heliopan E 1000" by Haarmann and Reimer,
   Cinoxate,
   DEA methoxycinnamate,
      - Diisopropyl methylcinnamate,
   Glyceryl ethylhexanoate dimethoxycinnamate.
Dibenzoylmethane derivatives:
   Butyl methoxydibenzoylmethane sold in particular under the trade name "Parsol 1789" by Hoffmann LaRoche, Isopropyldibenzoylmethane.
β,β-Diphenylacrylate derivatives:
   Octocrylene sold in particular under the trade name "Uvinul N539" by BASF,
   Etocrylene sold in particular under the trade name "Uvinul N35" by BASF.
Benzophenone derivatives:
   Benzophenone-1 sold under the trade name "Uvinul 400" by BASF,
   Benzophenone-2 sold under the trade name "Uvinul D50" by BASF,
   Benzophenone-3 or Oxybenzone sold under the trade name "Uvinul M40" by BASF,
   Benzophenone-4 sold under the trade name "Uvinul MS40" by BASF,
   Benzophenone-5,
   Benzophenone-6 sold under the trade name "Helisorb 11" by Norquay,
   Benzophenone-8 sold under the trade name "Spectra-Sorb UV-24" by American Cyanamid,
   Benzophenone-9 sold under the trade name "Uvinul DS-49" by BASF,
   Benzophenone-12
   n-hexyl 2-(4-diethylamino-2-hydroxybenzoyl)benzoate.
Benzylidenecamphor derivatives:
   3-Benzylidenecamphor manufactured under the name "Mexoryl SD" by Chimex,
   4-Methylbenzylidenecamphor sold under the name "Eusolex 6300" by Merck,
   Benzylidenecamphorsulfonic acid manufactured under the name "Mexoryl SL" by Chimex,
   Camphor benzalkonium methosulfate manufactured under the name "Mexoryl SO" by Chimex,
   Terephthalylidenedicamphorsulfonic acid manufactured under the name "Mexoryl SX" by Chimex,
   Polyacrylamidomethylbenzylidenecamphor manufactured under the name "Mexoryl SW" by Chimex.
Phenylbenzimidazole derivatives:
   Phenylbenzimidazolesulfonic acid sold in particular under the trade name "Eusolex 232" by Merck,
   Disodium phenyldibenzimidazoletetrasulfonate sold under the trade name "Neo Heliopan AP" by Haarmann and Reimer.
Phenylbenzotriazole derivatives:
   Drometrizole trisiloxane sold under the name "Silatrizole" by Rhodia Chimie,
   Methylenebis(benzotriazolyl)tetramethylbutylphenol sold in solid form under the trade name "MIXXIM BB/100" by Fairmount Chemical, or in micronized form as an aqueous dispersion under the trade name "Tinosorb M" by Ciba Specialty Chemicals.
Anthranilic derivatives:
   Menthyl anthranilate sold under the trade name "Neo Heliopan MA" by Haarmann and Reimer.
Imidazoline derivatives:
   Ethylhexyldimethoxybenzylidenedioxoimidazoline propionate.
Benzalmalonate derivatives:
   Polyorganosiloxane containing benzalmalonate functions, for instance Polysilicone-15, sold under the trade name "Parsol SLX" by Hoffmann LaRoche
4,4-Diarylbutadiene derivatives:
   - 1,1-Dicarboxy(2,2'-dimethylpropyl)-4,4-diphenyl-butadiene,
Benzoxazole derivatives:
   2,4-bis[5-1(dimethylpropyl)benzoxazol-2-yl(4-phenyl)imino]-6-(2-ethylhexyl)imino-1,3,5-triazine sold under the name Uvasorb K2A by Sigma 3V
and mixtures thereof.

The preferred additional organic UV-screening agents are chosen from:
Homosalate,
   Ethylhexyl salicylate,
   Ethylhexyl methoxycinnamate,
   Butylmethoxydibenzoylmethane,
   Octocrylene,
   Phenylbenzimidazolesulfonic acid,
   Benzophenone-3,
   Benzophenone-4,
   Benzophenone-5,
   n-Hexyl 2-(4-diethylamino-2-hydroxybenzoyl)benzoate,
   4-Methylbenzylidenecamphor,
   Terephthalylidenedicamphorsulfonic acid,
   Disodium phenyldibenzimidazoletetrasulfonate,
   Methylenebis(benzotriazolyl)tetramethylbutylphenol,
   Drometrizole trisiloxane,
   Polysilicone-15,
   1,1-Dicarboxy(2,2'-dimethylpropyl)-4,4-diphenyl-butadiene,
   2,4-Bis[5-1(dimethylpropyl)benzoxazol-2-yl(4-phenyl)imino]-6-(2-ethylhexyl)imino-1,3,5-triazine,
and mixtures thereof.

The additional mineral photoprotective agents are chosen from pigments and even more preferably nanopigments (mean size of the primary particles: generally between 5 nm and 100 nm and preferably between 10 nm and 50 nm) of treated or untreated metal oxides such as, for example, nanopigments of titanium oxide (amorphous or crystallized in rutile and/or anatase form), or of iron oxide, zinc oxide, zirconium oxide or cerium oxide.

The treated nanopigments are pigments that have undergone one or more surface treatments of chemical, electronic, mechanochemical and/or mechanical nature with compounds as described, for example, in Cosmetics & Toiletries, February 1990, Vol. 105, pp. 53-64, such as amino acids, beeswax, fatty acids, fatty alcohols, anionic surfactants, lecithins, sodium, potassium, zinc, iron or aluminium salts of fatty acids, metal (titanium or aluminium) alkoxides, polyethylene, silicones, proteins (collagen or elastin), alkanolamines, silicon oxides, metal oxides, sodium hexametaphosphate, alumina or glycerol.

The treated nanopigments may more particularly be titanium oxides treated with:
- silica and alumina, such as the products "Microtitanium Dioxide MT 500 SA" and "Microtitanium dioxide MT 100 SA" from the company Tayca, and the products "Tioveil Fin", "Tioveil OP", "Tioveil MOTG" and "Tioveil IPM" from the company Tioxide,
- alumina and aluminium stearate, such as the product "Microtitanium Dioxide MT 100 T" from the company Tayca,
- alumina and aluminium laurate, such as the product "Microtitanium Dioxide MT 100 S" from the company Tayca,
- iron oxides and iron stearate, such as the product "Microtitanium Dioxide MT 100 F" from the company Tayca,
- silica, alumina and silicone, such as the products "Microtitanium Dioxide MT 100 SAS", "Microtitanium Dioxide MT 600 SAS" and "Microtitanium Dioxide MT 500 SAS" from the company Tayca,
- sodium hexametaphosphate, such as the product "Microtitanium Dioxide MT 150 W" from the company Tayca,
- octyltrimethoxysilane, such as the product "T-805" from the company Degussa,
- alumina and stearic acid, such as the product "UVT-M160" from the company Kemira,
- alumina and glycerol, such as the product "UVT-M212" from the company Kemira,
- alumina and silicone, such as the product "UVT-M262" from the company Kemira.

Other titanium oxide nanopigments treated with a silicone are preferably TiO₂ treated with octyltrimethylsilane and for which the mean size of the elementary particles is between 25 and 40 nm, such as the product sold under the trade name "T 805" by the company Degussa Silices, TiO₂ treated with a polydimethylsiloxane and for which the mean size of the elementary particles is 21 nm, such as the product sold under the trade name "70250 Cardre UF TiO2SI3" by the company Cardre, anatase/rutile TiO₂ treated with a polydimethylhydrogenosiloxane and for which the mean size of the elementary particles is 25 nm, such as the product sold under the trade name "Microtitanium Dioxide USP Grade Hydrophobic" by the company Color Techniques.

The uncoated titanium oxide nanopigments are sold, for example, by the company Tayca under the trade names "Microtitanium Dioxide MT 500 B" or "Microtitanium Dioxide MT 600 B", by the company Degussa under the name "P 25", by the company Wackher under the name "Oxyde de titane transparent PW", by the company Miyoshi Kasei under the name "UFTR", by the company Tomen under the name "ITS" and by the company Tioxide under the name "Tioveil AQ".

The uncoated zinc oxide nanopigments are, for example:
- those sold under the name "Z-Cote" by the company Sunsmart;
- those sold under the name "Nanox" by the company Elementis;
- those sold under the name "Nanogard WCD 2025" by the company Nanophase Technologies.

The coated zinc oxide nanopigments are, for example:
- those sold under the name "Zinc Oxide CS-5" by the company Toshibi (ZnO coated with polymethylhydrogenosiloxane);
- those sold under the name "Nanogard Zinc Oxide FN" by the company Nanophase Technologies (as a 40% dispersion in Finsolv TN, C₁₂-C₁₅ alkyl benzoate);
- those sold under the name "Daitopersion ZN-30" and "Daitopersion ZN-50" by the company Daito (dispersions in cyclopolymethylsiloxane/oxyethylenated polydimethylsiloxane, containing 30% or 50% of nanozinc oxides coated with silica and polymethylhydrogenosiloxane);
- those sold under the name "NFD Ultrafine ZNO" by the company Daikin (ZnO coated with perfluoroalkyl phosphate and copolymer based on perfluoroalkylethyl as a dispersion in cyclopentasiloxane);
- those sold under the name "SPD-Z1" by the company Shin-Etsu (ZnO coated with silicone-grafted acrylic polymer, dispersed in cyclodimethylsiloxane);
- those sold under the name "Escalol Z100" by the company ISP (alumina-treated ZnO dispersed in an ethylhexyl methoxycinnamate/PVP-hexadecene copolymer/methicone mixture);
- those sold under the name "Fuji ZNO-SMS-10" by the company Fuji Pigment (ZnO coated with silica and polymethylsilsesquioxane);
- those sold under the name "Nanox Gel TN" by the company Elementis (ZnO dispersed at a concentration of 55% in C₁₂-C₁₅ alkyl benzoate with hydroxystearic acid polycondensate).

The uncoated cerium oxide nanopigments are sold under the name "Colloidal Cerium Oxide" by the company Rhone-Poulenc.

The uncoated iron oxide nanopigments are sold, for example, by the company Arnaud under the names "Nanogard WCD 2002 (FE 45B)" and "Nanogard Iron FE 45 BL AQ", "Nanogard FE 45R AQ", "Nanogard WCD 2006 (FE 45R)" or by the company Mitsubishi under the name "TY-220".

The coated iron oxide nanopigments are sold, for example, by the company Arnaud under the names "Nanogard WCD 2008 (FE 45B FN)", "Nanogard WCD 2009 (FE 45B 556)", "Nanogard FE 45 BL 345" and "Nanogard FE 45 BL" or by the company BASF under the name "Transparent Iron Oxide".

Mention may also be made of mixtures of metal oxides, especially of titanium dioxide and of cerium dioxide, including the silica-coated equal-weight mixture of titanium dioxide and of cerium dioxide, sold by the company Ikeda under the name "Sunveil A", and also the alumina, silica and silicone-coated mixture of titanium dioxide and of zinc dioxide, such as the product "M 261" sold by the company Kemira, or the alumina, silica and glycerol-coated mixture of titanium dioxide and of zinc dioxide, such as the product "M 211" sold by the company Kemira.

The nanopigments may be introduced into the compositions according to the invention in unmodified form or in the form of pigmentary paste, i.e. as a mixture with a dispersant, as described, for example, in document GB-A-2 206 339.

The additional photoprotective agents are generally present in the compositions according to the invention in proportions ranging from 0.01% to 20% by weight relative to the total weight of the composition and preferably ranging from 0.1% to 10% by weight relative to the total weight of the composition.

The compositions according to the invention may also contain agents for artificially tanning and/or browning the skin (self-tanning agents) and more particularly dihydroxyacetone (DHA). They are preferably present in amounts ranging from 0.1% to 10% by weight relative to the total weight of the composition.

The compositions in accordance with the present invention may also comprise standard cosmetic adjuvants chosen especially from fatty substances, organic solvents, ionic or nonionic, hydrophilic or lipophilic thickeners, softeners, humectants, opacifiers, stabilizers, emollients, silicones, antifoams, fragrances, preserving agents, anionic, cationic, nonionic, zwitterionic or amphoteric surfactants, active agents, fillers, polymers, propellants, acidifying or basifying agents or any other ingredient usually used in cosmetics and/or dermatology.

The fatty substances may consist of an oil or a wax or mixtures thereof. The term "oil" means a compound that is liquid at room temperature. The term "wax" means a compound that is solid or substantially solid at room temperature and whose melting point is generally greater than 35°C.

Oils that may be mentioned include mineral oils (paraffin); plant oils (sweet almond oil, macadamia oil, grapeseed oil or jojoba oil); synthetic oils, for instance perhydrosqualene, fatty alcohols, fatty acids or fatty esters (for instance the C₁₂-C₁₅ alkyl benzoate sold under the trade name "Finsolv TN" by the company Witco, octyl palmitate, isopropyl lanolate and triglycerides, including capric/caprylic acid triglycerides), oxyethylenated or oxypropylenated fatty esters and ethers; silicone oils (cyclomethicone and polydimethylsiloxanes, or PDMS) or fluoro oils, and polyalkylenes.

Waxy compounds that may be mentioned include paraffin, carnauba wax, beeswax and hydrogenated castor oil.

Among the organic solvents that may be mentioned are lower alcohols and polyols. These polyols may be chosen from glycols and glycol ethers, for instance ethylene glycol, propylene glycol, butylene glycol, dipropylene glycol or diethylene glycol.

Hydrophilic thickeners that may be mentioned include carboxyvinyl polymers such as the Carbopol products (carbomers) and the Pemulen products (acrylate/C10-C30-alkylacrylate copolymer); polyacrylamides, for instance the crosslinked copolymers sold under the names Sepigel 305 (CTFA name: polyacrylamide/C13-14 isoparaffin/Laureth 7) or Simulgel 600 (CTFA name: acrylamide/sodium acryloyldimethyltaurate copolymer/isohexadecane/polysorbate 80) by the company SEPPIC; 2-acrylamido-2-methylpropanesulfonic acid polymers and copolymers, which are optionally crosslinked and/or neutralized, for instance the poly(2-acrylamido-2-methylpropanesulfonic acid) sold by the company Hoechst under the trade name "Hostacerin AMPS" (CTFA name: ammonium polyacryldimethyltauramide); cellulose-based derivatives such as hydroxyethylcellulose; polysaccharides and especially gums such as xanthan gum; and mixtures thereof.

Lipophilic thickeners that may be mentioned include modified clays such as hectorite and its derivatives, for instance the products sold under the name Bentone.

Among the active agents that may be mentioned are:
- antipollution agents and/or free-radical scavengers;
- depigmenting agents and/or propigmenting agents;
- antiglycation agents;
- NO-synthase inhibitors;
- agents for stimulating the synthesis of dermal or epidermal macromolecules and/or for preventing their degradation;
- agents for stimulating fibroblast proliferation;
- agents for stimulating keratinocyte proliferation;
- muscle relaxants;
- tensioning agents;
- desquamating agents;
- moisturizers;
- anti-inflammatory agents;
- agents acting on the energy metabolism of cells;
- insect repellants;
- substance P or CGRP antagonists.

Needless to say, a person skilled in the art will take care to select the optional additional compound(s) mentioned above and/or the amounts thereof such that the advantageous properties intrinsically associated with the compositions in accordance with the invention are not, or are not substantially, adversely affected by the envisaged addition(s).

The compositions according to the invention may be prepared according to techniques that are well known to those skilled in the art, in particular those intended for the preparation of emulsions of oil-in-water or water-in-oil type. They may be in particular in the form of a simple or complex emulsion (O/W, W/O, O/W/O or W/O/W emulsion) such as a cream or a milk, in the form of a gel or a cream-gel, or in the form of a lotion, a powder or a solid stick, and may optionally be packaged as an aerosol and may be in the form of a mousse or a spray.

The compositions according to the invention are preferably in the form of an oil-in-water or water-in-oil emulsion.

The emulsions generally contain at least one emulsifier chosen from amphoteric, anionic, cationic and nonionic emulsifiers, which are used alone or as a mixture. The emulsifiers are appropriately chosen according to the emulsion to be obtained (W/O or O/W).

As emulsifying surfactants that may be used for the preparation of the W/O emulsions, examples that may be mentioned include sorbitan, glycerol or sugar alkyl esters or ethers; silicone surfactants, for instance dimethicone copolyols, such as the mixture of cyclomethicone and of dimethicone copolyol, sold under the name "DC 5225 C" by the company Dow Corning, and alkyldimethicone copolyols such as laurylmethicone copolyol sold under the name "Dow Corning 5200 Formulation Aid" by the company Dow Corning; cetyldimethicone copolyol, such as the product sold under the name Abil EM 90R by the company Goldschmidt, and the mixture of cetyldimethicone copolyol, of polyglyceryl isostearate (4 mol) and of hexyl laurate, sold under the name Abil WE 09 by the company Goldschmidt. One or more co-emulsifiers may also be added thereto, which may be chosen advantageously from the group comprising polyol alkyl esters. Polyol alkyl esters that may especially be mentioned include glycerol and/or sorbitan esters, for example polyglyceryl isostearate, such as the product sold under the name Isolan GI 34 by the company Goldschmidt, sorbitan isostearate, such as the product sold under the name Arlacel 987 by the company ICI, sorbitan glyceryl isostearate, such as the product sold under the name Arlacel 986 by the company ICI, and mixtures thereof.

For the O/W emulsions, examples of emulsifiers that may be mentioned include nonionic emulsifiers such as oxyalkylenated (more particularly polyoxyethylenated) fatty acid esters of glycerol; oxyalkylenated fatty acid esters of sorbitan; oxyalkylenated (oxyethylenated and/or oxypropylenated) fatty acid esters; oxyalkylenated (oxyethylenated and/or oxypropylenated) fatty alkyl ethers; sugar esters, for instance sucrose stearate; fatty alkyl ethers of sugars, especially alkylpolyglucosides (APG) such as decylglucoside and laurylglucoside sold, for example, by the company Henkel under the respective names Plantaren 2000 and Plantaren 1200, cetostearyl glucoside optionally as a mixture with cetostearyl alcohol, sold, for example, under the name Montanov 68 by the company SEPPIC, under the name Tegocare CG90 by the company Goldschmidt and under the name Emulgade KE3302 by the company Henkel, and also arachidyl glucoside, for example in the form of a mixture of arachidyl alcohol, behenyl alcohol and arachidyl glucoside, sold under the name Montanov 202 by the company SEPPIC. According to one particular embodiment of the invention, the mixture of the alkylpolyglucoside as defined above with the corresponding fatty alcohol may be in the form of a self-emulsifying composition as described, for example, in document WO-A-92/06778.

When it is an emulsion, the aqueous phase of this emulsion may comprise a nonionic vesicular dispersion prepared according to known processes (Bangham, Standish and Watkins, J. Mol. Biol. 13, 238 (1965), FR 2 315 991 and FR 2 416 008).

The compositions according to the invention find their application in a large number of treatments, especially cosmetic treatments, of the skin, the lips and the hair, including the scalp, especially for protecting and/or caring for the skin, the lips and/or the hair, and/or for making up the skin and/or the lips.

Another subject of the present invention consists of the use of the compositions according to the invention as defined above for the manufacture of products for cosmetically treating the skin, the lips, the nails, the hair, the eyelashes, the eyebrows and/or the scalp, especially care products and makeup products.

The cosmetic compositions according to the invention may be used, for example, as care products and/or antisun products for the face and/or the body, of liquid to semi-liquid consistency, such as milks, more or less rich creams, cream-gels and pastes. They may optionally be packaged as an aerosol and may be in the form of mousses or sprays.

The compositions according to the invention in the form of vaporizable fluid lotions in accordance with the invention are applied to the skin or the hair in the form of fine particles by means of pressurization devices. The devices in accordance with the invention are well known to those skilled in the art and include non-aerosol pumps or "atomizers", aerosol containers comprising a propellant and also aerosol pumps using compressed air as propellant. The latter pumps are described in patents US 4 077 441 and US 4 850 517 (forming an integral part of the content of the description).

The compositions packaged in aerosol form in accordance with the invention generally contain conventional propellants, for instance hydrofluoro compounds, dichlorodifluoromethane, difluoroethane, dimethyl ether, isobutane, n-butane, propane or trichlorofluoromethane. They are preferably present in amounts ranging from 15% to 50% by weight relative to the total weight of the composition.

Concrete but in no way limiting examples illustrating the invention will now be given.

The antisun formulations below were prepared; the amounts are indicated as weight percentages:

| **Composition** | **Example 1** | **Example 2** |
|---|---|---|
| **PHASE A** | | |
| Polydimethylsiloxane | 0.5 | 0.5 |
| Preserving agents | 1.0 | 1.0 |
| Stearic acid | 1.5 | 1.5 |
| Glyceryl monostearate/ PEG stearate mixture (100 EO) | 1.0 | 1.0 |
| Cetylstearyl glucoside/cetylstearyl alcohol mixture | 2.0 | 2.0 |
| Cetyl alcohol | 0.5 | 0.5 |
| N-Lauroyl isopropyl sarcosinate (Eldew SL-205 - Ajinomoto) | 10 | 10 |
| Butylphthalimide/isopropylphthalimide eutectic mixture | 10 | 10 |
| Bis(ethylhexyloxyphenol)methoxyphenyltriazine (Tinosorb S from Ciba Geigy) | 5 | - |
| Ethylhexyl Triazone (Uvinul T150 from BASF) | - | 5 |

| **PHASE B** | | |
|---|---|---|
| Deionized water | qs 100 | qs 100 |
| Sequestering agent | 0.1 | 0.1 |
| Glycerol | 5.0 | 5.0 |
| Xanthan gum | 0.2 | 0.2 |
| Monocetyl phosphate | 1.0 | 1.0 |

| **PHASE C** | | |
|---|---|---|
| Isohexadecane | 1.0 | 1.0 |
| Acrylic acid/stearyl methacrylate copolymer | 0.2 | 0.2 |
| Triethanolamine | qs | qs |

The aqueous phase (Phase B) containing all of its ingredients is heated to 80°C in a water bath. The fatty phase (Phase A) containing all of its ingredients is heated to 80°C in a water bath. A is emulsified in B with stirring of rotor-stator type (machine from the company Moritz). Phase C is incorporated and the mixture is allowed to cool to room temperature with moderate stirring. The triethanolamine is introduced so as to adjust the pH to the desired value at the end of manufacture.

## Claims

1. Photoprotective cosmetic or dermatological composition, **characterized in that** it comprises:
a) at least one 1,3,5-triazine derivative and
b) at least one oil containing in its structure at least one amide unit and
c) at least one eutectic mixture of n-butylphthalimide and of isopropylphthalimide.

2. Composition according to Claim 1, in which the oil containing in its structure at least one amide unit is chosen from the compounds of formula **(1a) or (1b)** below: 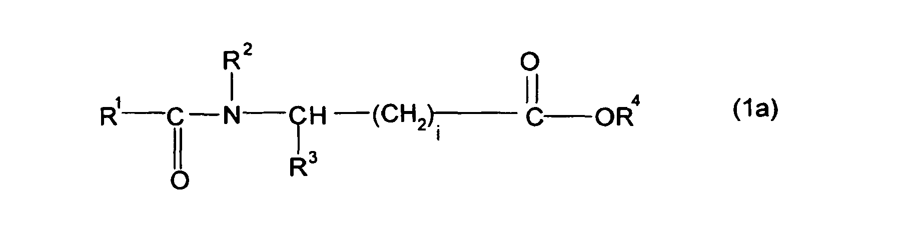 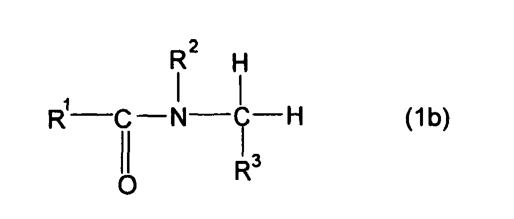 in which:
- the radical R¹ represents an optionally functionalized, aliphatic, cycloaliphatic or cyclic, saturated or unsaturated monovalent hydrocarbon-based radical containing from 1 to 30 carbon atoms and preferably from 1 to 22 carbon atoms, limits inclusive;
- the radicals R², R³ and R⁴, which may be identical or different, represent optionally functionalized, aliphatic, cycloaliphatic or cyclic, saturated or unsaturated monovalent hydrocarbon-based radicals containing from 1 to 30 carbon atoms and preferably from 1 to 22 carbon atoms, limits inclusive;
- i is an integer from 0 to 2;

3. Composition according to Claim 2, in which the oil containing in its structure at least one amide unit of formula (1a) or (1b) is chosen from those in which:
R¹ represents a linear or branched C₁-C₂₂ alkyl radical; a linear or branched C₂-C₂₂ alkenyl radical; an aryl radical;
R² represents a hydrogen atom or a linear or branched C₁-C₆ alkyl group;
R³ represents a hydrogen atom or a linear or branched C₁-C₆ alkyl group;
R⁴ represents a linear or branched C₁-C₁₀ alkyl radical or a linear or branched C₂-C₁₀ alkenyl radical or a sterol residue.

4. Composition according to Claim 3, in which the oil containing in its structure at least one amide unit of formula (1a) or (1b) is chosen from:
ethyl N-acetyl-N-butylaminopropionate, having the following formula: 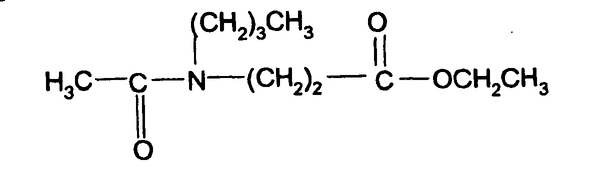
isopropyl N-lauroylsarcosinate of formula: 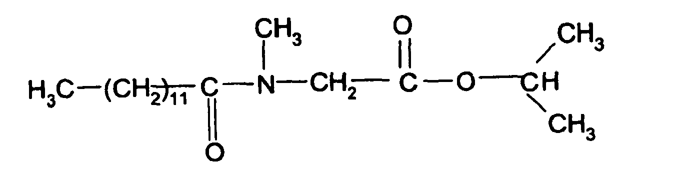
N,N-diethyltoluamide of formula:

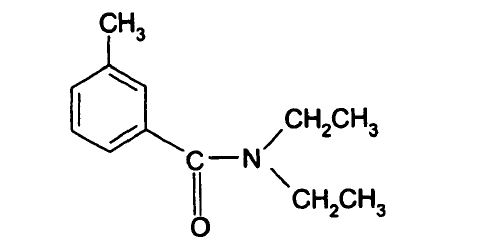

5. Composition according to any one of Claims 1 to 4, in which the oil(s) containing in their structure at least one amide function is (are) present in concentrations preferably ranging from 0.1% to 40% by weight and more preferably from 1% to 20% by weight relative to the total weight of the composition.

6. Composition according to any one of Claims 1 to 5, in which the eutectic mixture consists of:
(a) 60% to 75% by weight of n-butylphthalimide of structure (2) below: 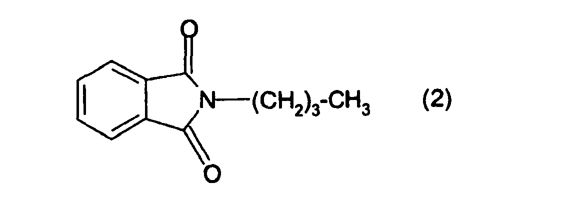
(b) 25% to 40% by weight of isopropylphthalimide in accordance with the invention, corresponding to structure (3) below:

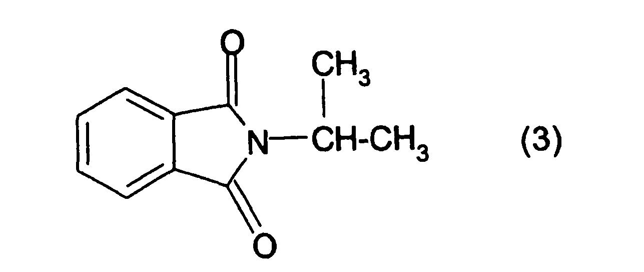

7. Composition according to any one of Claims 1 to 6, in which the eutectic mixture is chosen from:
n-butylphthalimide/isopropylphthalimide (60/40% by weight)
n-butylphthalimide/isopropylphthalimide (62/38% by weight)
n-butylphthalimide/isopropylphthalimide (65/35% by weight)
n-butylphthalimide/isopropylphthalimide (70/30% by weight)
n-butylphthalimide/isopropylphthalimide (75/25% by weight).

8. Composition according to any one of Claims 1 to 7, in which the eutectic mixture is present in concentrations ranging from 0.1% to 50% by weight and more preferably from 1% to 30% by weight relative to the total weight of the composition.

9. Composition according to any one of Claims 1 to 8, in which the 1,3,5-triazine derivative corresponds to formula (I) below: 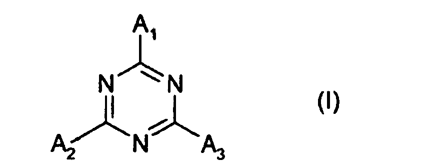 in which the radicals A₁, A₂ and A₃, which may be identical or different, are chosen from the groups of formulae (II) to (IX) below: 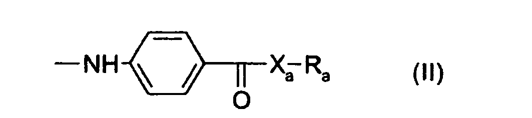 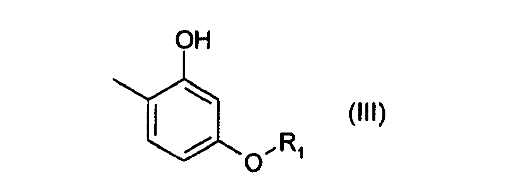 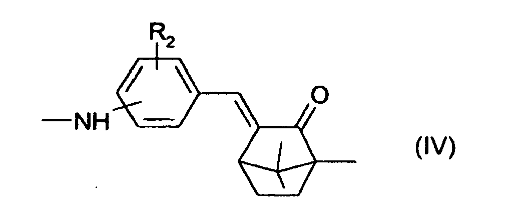 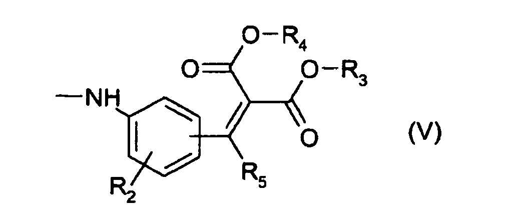 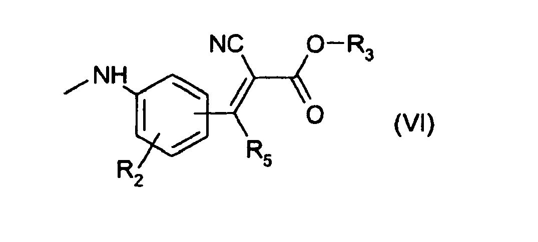 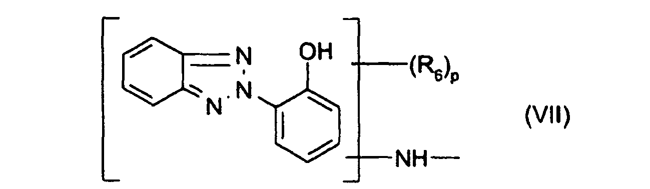 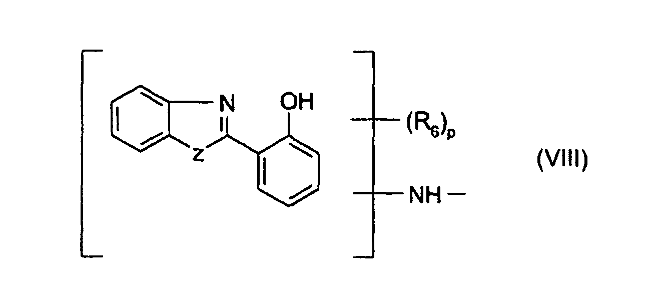 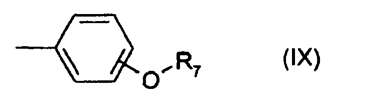 in which:
- Xₐ (each of the groups Xₐ may be identical or different) represents oxygen or -NH-;
- Rₐ (each of the groups Rₐ may be identical or different) is chosen from hydrogen; an alkali metal; an ammonium radical optionally substituted with one or more linear or branched C₁-C₁₈ alkyl or linear or branched C₁-C₁₈ hydroxyalkyl radicals; a linear or branched C₁-C₁₈ and preferably C₆-C₁₂ alkyl radical; a C₅-C₁₂ cycloalkyl radical optionally substituted with one or more C₁-C₄ alkyl radicals; a polyoxyethylenated radical comprising from 1 to 6 ethylene oxide units, the terminal OH group of which is methylated; a radical of formula (X), (XI) or (XII) below: 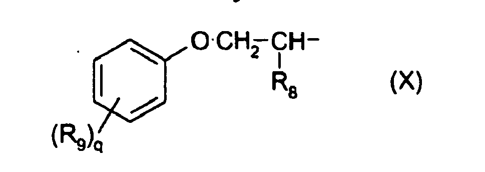 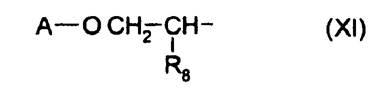 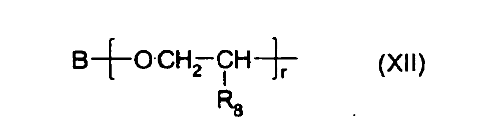
in which:
- R₈ is hydrogen or a methyl radical;
- R₉ is a C₁-C₉ alkyl radical;
- q is an integer equal to 0; 1; 2; 3;
- r is an integer equal to 1; 2; 3; 4; 5; 6; 7; 8; 9; 10;
- A is a C₄-C₈ alkyl radical or a C₅-C₈ cycloalkyl radical;
- B is chosen from: a linear or branched C₁-C₈ alkyl radical; a C₅-C₈ cycloalkyl radical; an aryl radical optionally substituted with one or more C₁-C₄ alkyl radicals;
- R₁ denotes a C₃-C₁₈ alkyl radical; a C₂-C₁₈ alkenyl radical; a residue of formula -CH₂-CH(OH)-CH₂-OT₁ in which T₁ is a hydrogen atom or a C₁-C₈ alkyl radical; a residue of formula (XIII) below: 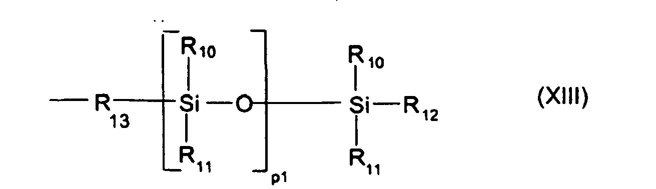
in which:
- R₁₃ denotes a covalent bond; a linear or branched C₁-C₄ alkyl radical or a radical of formula -Cₘ₁H₂ₘ₁-O- in which m₁ is an integer equal to 1; 2; 3; 4;
- p₁ is an integer equal to 0; 1; 2; 3; 4; 5;
- the radicals R₁₀, R₁₁ and R₁₂, which may be identical or different, denote a C₁-C₁₈ alkyl radical; a C₁-C₁₈ alkoxy radical or a radical of formula: 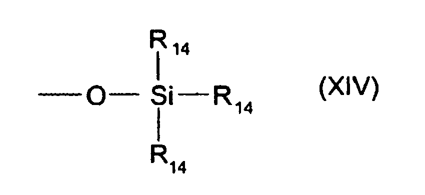
in which R₁₄ is a C₁-C₅ alkyl radical;
- R₂ denotes a hydrogen atom, a linear or branched C₁-C₄ alkyl radical or a C₁-C₄ alkoxy radical;
- R₃ and R₄, which may be identical or different, denote a linear or branched C₁-C₂₀ alkyl radical;
- R₅ represents a hydrogen atom or a phenyl radical optionally substituted with a halogen or with a C₁-C₄ alkyl radical or with a C₁-C₄ alkoxy radical;
- R₆ is a linear or branched C₁-C₈ alkyl radical or a C₁-C₃ alkoxy radical, it being understood that, in the latter case, two adjacent radicals R₆ on the same aromatic nucleus can together form an alkylidenedioxy group in which the alkylidene group contains 1 or 2 carbon atoms, OH, NHCOCH₃ or NH₂,
- R₇ denotes a hydrogen atom, a C₁-C₁₀ alkyl radical, a radical of formula: -(CH₂CHR₅-O)ₙ₁R₈ in which n₁ is a number from 1 to 16, or a radical of structure -CH₂-CH-(OH)-CH₂OT₁ with R₈ and T₁ having the same meaning as indicated above,
- Z represents oxygen, sulfur, -NH- or -NR₃- with R₃ representing a linear or branched C₁-C₂₀ alkyl radical;
- p is 0, 1, 2 or 3,
A₁ can also be a halogen, a radical -N (R₃)₂, the two radicals R₃ together possibly forming a ring of 4 or 5 carbon atoms, or a group -OR₃, R₃ having the same definition as above.

10. Composition according to Claim 9, in which the 1,3,5-triazine derivative corresponds to formula (I) in which A₁, A₂ and A₃ are of formula (II) and have all of the following characteristics:
- one of the groups Xₐ-Rₐ represents a radical-NH-Rₐ with Rₐ chosen from: a C₅-C₁₂ cycloalkyl radical optionally substituted with one or more C₁-C₄ alkyl radicals; a radical of formula (X), (XI) or (XII) above in which:
- B is a C₁-C₄ alkyl radical;
- R₉ is a methyl radical;
- the other two groups Xₐ-Rₐ represent a radical -O-Rₐ with Rₐ, which may be identical or different, chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a radical of formula (X), (XI) or (XII) above in which:
- B is a C₁-C₄ alkyl radical;
- R₉ is a methyl radical.

11. Composition according to Claim 9, in which the 1,3,5-triazine derivative corresponds to formula (I) in which A₁, A₂ and A₃ are of formula (II) and have all of the following characteristics:
- one or two groups Xₐ-Rₐ represent a radical -NH-Rₐ, with Rₐ chosen from: a linear or branched C₁-C₁₈ alkyl radical; a C₅-C₁₂ cycloalkyl radical optionally substituted with one or more C₁-C₄ alkyl radicals; a radical of formula (X), (XI) or (XII) above in which:
- B is a C₁-C₄ alkyl radical;
- R₉ is a methyl radical;
the other or the other two group(s) Xₐ-Rₐ being a radical -O-Rₐ with Rₐ, which may be identical or different, chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a radical of formula (X), (XI) or (XII) above in which:
- B is a C₁-C₄ alkyl radical;
- R₉ is a methyl radical.

12. Composition according to Claim 11, in which the 1,3,5-triazine derivative is 2-[(p-(tert-butylamido)anilino]-4,6-bis[(p-(2'-ethylhexyl-1'-oxycarbonyl)anilino]-1,3,5-triazine of the following formula: 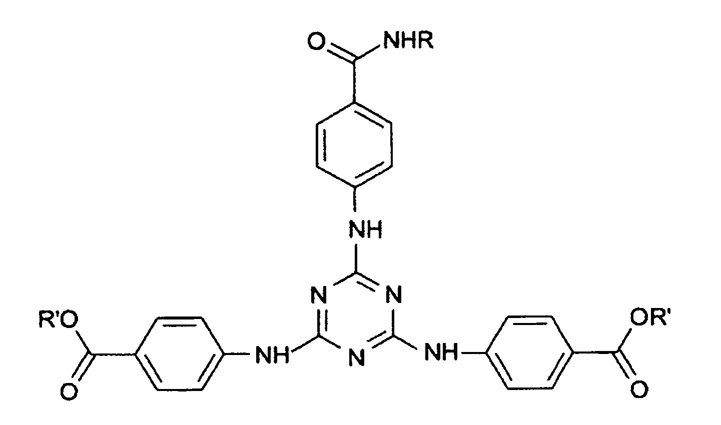 in which R' denotes a 2-ethylhexyl radical and R denotes a tert-butyl radical.

13. Composition according to Claim 9, in which the 1,3,5-triazine derivative corresponds to formula (I) in which A₁, A₂ and A₃ are of formula (II) and have all of the following characteristics:
- Xₐ are identical and represent oxygen;
- Rₐ, which may be identical or different, represent a C₆-C₁₂ alkyl radical or a polyoxyethylenated radical comprising from 1 to 6 ethylene oxide units, the terminal OH group of which is methylated.

14. Composition according to Claim 13, in which the 1,3,5-triazine derivative is 2,4,6-tris[p(2'-ethylhexyl-1'-oxycarbonyl)anilino]-1,3,5-triazine of the following formula: 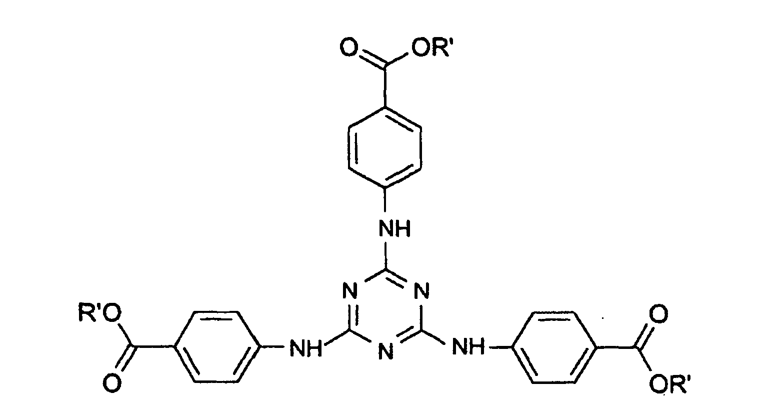 in which R' denotes a 2-ethylhexyl radical.

15. Composition according to Claim 9, in which the 1,3,5-triazine derivative corresponds to formula (I) in which A₁ and A₂ are of formula (III) and A₃ is of formula (IX) and have all of the following characteristics: R₁, which may be identical or different, denote a C₃-C₁₈ alkyl radical; a C₂-C₁₈ alkenyl radical or a residue of formula -CH₂-CH(OH)-CH₂-OT₁ in which T₁ is a hydrogen atom or a C₁-C₈ alkyl radical; R₇ denotes a hydrogen atom or a C₁-C₁₀ alkyl radical.

16. Composition according to Claim 15, in which the 1,3,5-triazine derivative is 2,4-bis{[4-2-ethylhexyloxy)]-2-hydroxy]phenyl)-6-(4-methoxyphenyl)-1,3,5-triazine of the following formula: 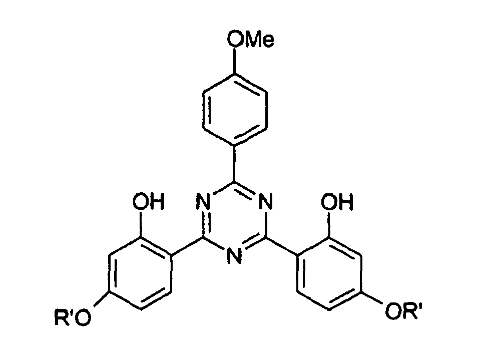 in which R' denotes a 2-ethylhexyl radical.

17. Composition according to Claim 9, in which the 1,3,5-triazine derivative corresponds to formula (I) in which A₁, A₂ and A₃ are of formulae (VII) to (XI).

18. Composition according to Claim 17, in which the 1,3,5-triazine derivative is chosen from:
2,4,6-tris(diisobutyl 4'-aminobenzalmalonate)-s-triazine,
2,4,6-tris(bis(2-ethylhexyl) 4'-aminobenzalmalonate)-s-triazine,
2,4,6-tris(bis(2-ethylhexyl) 9'-aminobenzalmalonate)-6-chloro-s-triazine,
2,4,6-tris(bis(2-ethylhexyl) 4'-aminobenzalmalonate)-6-(2-ethylhexyl 4'-aminobenzoate)-s-triazine,
2,9,6-tris(diisobutyl 4'-aminobenzalmalonate)-6-butoxy-s-triazine,
2,4,6-tris(diisobutyl 4'-aminobenzalmalonate)-6-(2-ethylhexylamino)-s-triazine,
2,4-bis(4'-aminobenzylidenecamphor)-6-(2-ethyl-hexylamino)-s-triazine,
2,4-bis(4'-aminobenzylidenecamphor)-6-(diisobutyl 4'-aminobenzalmalonate)-s-triazine,
2,4,6-tris(diethyl 4'-aminobenzalmalonate)-s-triazine,
2,4,6-tris(diisopropyl 4'-aminobenzalmalonate)-s-triazine,
2,4,6-tris(dimethyl 4'-aminobenzalmalonate)-s-triazine,
2,4,6-tris(ethyl α-cyano-4-aminocinnamate)-s-triazine,
2,4,6-tris[(3'-benzotriazol-2-yl-2'-hydroxy-5'-methyl)phenylamino]-s-triazine,
2,4,6-tris[(3'-benzotriazol-2-yl-2'-hydroxy-5'-tert-octyl)phenylamino]-s-triazine.

19. Composition according/to any one of Claims 1 to 18, in which the 1,3,5-triazine derivative(s) is (are) present in concentrations ranging from 0.05% to 15% and preferably from 0.1% to 10% by weight relative to the total weight of the said composition.

20. Composition according to any one of Claims 1 to 19, also containing at least one additional organic photoprotective agent and/or at least one additional mineral photoprotective agent, which is water-soluble, liposoluble or insoluble in the cosmetic solvents commonly used.

21. Composition according to Claim 20, in which the additional organic photoprotective agents are chosen from anthranilates; dibenzoylmethane derivatives; cinnamic derivatives; salicylic derivatives; camphor derivatives; benzophenone derivatives; β, β'-diphenylacrylate derivatives; benzotriazole derivatives; benzalmalonate derivatives; benzimidazole derivatives; imidazolines; bis-benzazolyl derivatives; p-aminobenzoic acid (PABA) derivatives; methylenebis(hydroxyphenylbenzotriazole) derivatives; benzoxazole derivatives; screening polymers and screening silicones; dimers derived from α-alkyl-styrene; 4,4-diarylbutadienes, and mixtures thereof.

22. Composition according to Claim 21, in which the additional organic screening agents are chosen from:
Homosalate,
Ethylhexyl salicylate,
Ethylhexyl methoxycinnamate,
Butylmethoxydibenzoylmethane,
Octocrylene,
Phenylbenzimidazolesulfonic acid,
Benzophenone-3,
Benzophenone-4,
Benzophenone-5,
n-Hexyl 2-(4-diethylamino-2-hydroxybenzoyl)benzoate,
4-Methylbenzylidenecamphor,
Terephthalylidenedicamphorsulfonic acid,
Disodium phenyldibenzimidazoletetrasulfonate,
Methylenebis(benzotriazolyl)tetramethylbutylphenol,
Drometrizole trisiloxane,
Polysilicone-15,
1,1-Dicarboxy(2,2'-dimethylpropyl)-4,4-diphenyl-butadiene,
2,4-Bis[5-1(dimethylpropyl)benzoxazol-2-yl(4-phenyl)imino]-6-(2-ethylhexyl)imino-1,3,5-triazine,
and mixtures thereof.

23. Composition according to Claim 20, in which the additional mineral photoprotective agents are chosen from coated or uncoated metal oxide pigments or nanopigments.

24. Composition according to Claim 23, in which the additional mineral screening agents are nanopigments of titanium oxide, which is amorphous or crystallized in rutile and/or anatase form, or of iron oxide, zinc oxide, zirconium oxide or cerium oxide.

25. Composition according to any one of Claims 1 to 24, **characterized in that** it also comprises at least one agent for artificially tanning and/or browning the skin.

26. Composition according to any one of Claims 1 to 25, **characterized in that** it also comprises at least one adjuvant chosen from fatty substances, organic solvents, ionic or nonionic, hydrophilic or lipophilic thickeners, softeners, humectants, opacifiers, stabilizers, emollients, silicones, antifoams, fragrances, preserving agents, anionic, cationic, nonionic, zwitterionic or amphoteric surfactants, active agents, fillers, polymers, propellants and acidifying or basifying agents.

27. Composition according to any one of Claims 1 to 26, **characterized in that** the combination of eutectic mixture of n-butylphthalimide and of isopropylphthalimide/amide-based oil is present in the composition in an amount that is sufficient for dissolving by itself all of the triazine screening agent present in the composition.

28. Use of a composition as defined in any one of Claims 1 to 27, for the manufacture of products for cosmetically treating the skin, the lips, the nails, the hair, the eyelashes, the eyebrows and/or the scalp.

29. Use of a composition as defined in any one of Claims 1 to 27, for the manufacture of products for caring for the skin, the lips, the nails, the hair and/or the scalp.

30. Use of a composition as defined in any one of Claims 1 to 27, for the manufacture of makeup products.

31. Use of a combination of (i) at least one oil containing in its structure at least one amide unit and (ii) at least one eutectic mixture of n-butylphthalimide and of isopropylphthalimide as defined in any one of the preceding claims, in a cosmetic or dermatological composition containing a 1,3,5-triazine derivative as defined in the preceding claims, to improve the sun protection factor, the cosmetic properties and/or the stability of this composition.

## Patentansprüche

1. Kosmetische oder dermatologische Lichtschutzzusammensetzung, **dadurch gekennzeichnet, dass** sie enthält:
a) mindestens ein 1,3,5-Triazinderivat und
b) mindestens ein Öl, das in seiner Struktur mindestens eine Amideinheit aufweist, und
c) mindestens ein eutektisches Gemisch von n-Butylphthalimid und Isopropylphthalimid.

2. Zusammensetzung nach Anspruch 1, wobei das Öl, das in seiner Struktur mindestens eine Amideinheit aufweist, unter den Verbindungen der folgenden Formel (1a) oder (1b) ausgewählt ist: 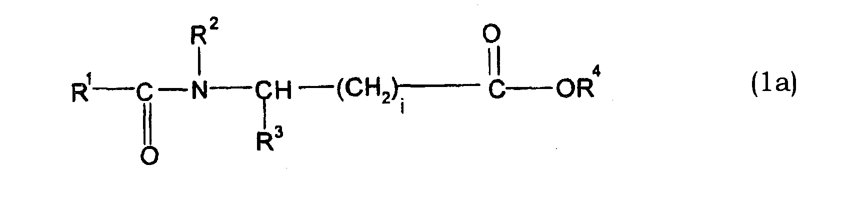 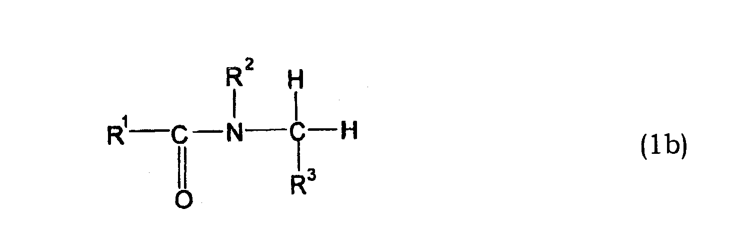 worin bedeuten:
- die Gruppe R¹ eine gegebenenfalls funktionalisierte, aliphatische, cycloaliphatische oder cyclische, gesättigte oder ungesättigte, einwertige Gruppe auf Kohlenwasserstoffbasis mit 1 bis 30 Kohlenstoffatomen und vorzugsweise 1 bis 22 Kohlenstoffatomen, wobei die Grenzen eingeschlossen sind;
- die Gruppen R², R³ und R⁴, die gleich oder verschieden sein können, gegebenenfalls funktionalisierte, aliphatische, cycloaliphatische oder cyclische, gesättigte oder ungesättigte, einwertige Gruppen auf Kohlenwasserstoffbasis mit 1 bis 30 Kohlenstoffatomen und vorzugsweise 1 bis 22 Kohlenstoffatomen, wobei die Grenzen eingeschlossen sind;
- i 0 oder eine ganze Zahl von 1 bis 2.

3. Zusammensetzung nach Anspruch 2, wobei das Öl der Formel (1a) oder (1b), das in seiner Struktur mindestens eine Amideinheit aufweist, unter den Verbindungen ausgewählt ist, worin bedeuten:
R¹ eine lineare oder verzweigte C₁₋₂₂-Alkylgruppe;
eine lineare oder verzweigte C₂₋₂₂-Alkenylgruppe;
eine Arylgruppe;
R² ein Wasserstoffatom oder eine lineare oder verzweigte C₁₋₆-Alkylgruppe;
R³ ein Wasserstoffatom oder eine lineare oder verzweigte C₁₋₆-Alkylgruppe;
R⁴ eine lineare oder verzweigte C₁₋₁₀-Alkylgruppe oder eine lineare oder verzweigte C₂₋₁₀-Alkenylgruppe oder einen Sterolrest.

4. Zusammensetzung nach Anspruch 3, wobei das Öl der Formel (Ia) oder (Ib), das in seiner Struktur mindestens eine Amideinheit aufweist, ausgewählt ist unter:
Ethyl-N-acetyl-N-butylaminopropionat der folgenden Formel: 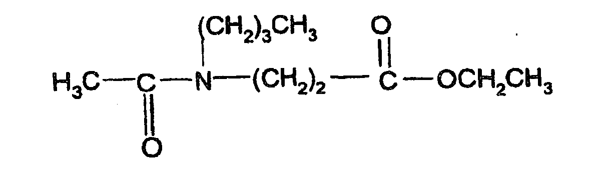
Isopropyl-N-lauroylsarcosionat der Formel: 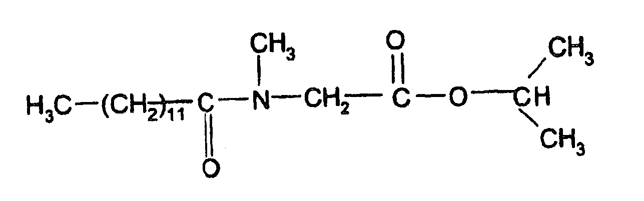
N,N-Diethyltoluamid der Formel:

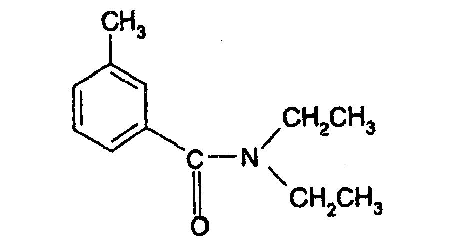

5. Zusammensetzung nach einem der Ansprüche 1 bis 4, wobei das oder die Öl(e), die in ihrer Struktur mindestens eine Amidfunktion aufweisen, in Konzentrationen von vorzugsweise 0,1 bis 40 Gew.-% und noch bevorzugter 1 bis 20 Gew.-%, bezogen auf das Gesamtgewicht der Zusammensetzung, enthalten ist (sind).

6. Zusammensetzung nach einem der Ansprüche 1 bis 5, wobei das eutektische Gemisch besteht aus:
(a) 60 bis 70 Gew.-% n-Butylphthalimid der folgenden Struktur (2): 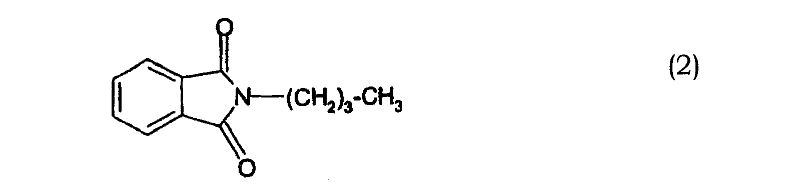
(b) 25 bis 40 Gew.-% Isopropylphthalimid gemäß der Erfindung der folgenden Struktur (3):

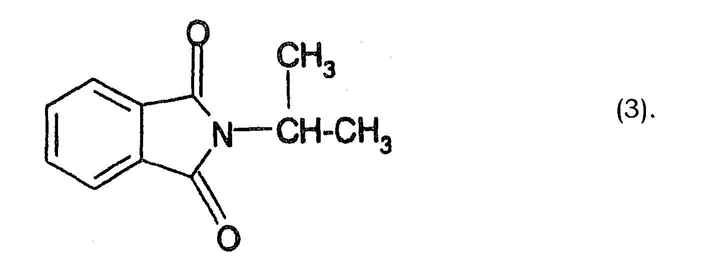

7. Zusammensetzung nach einem der Ansprüche 1 bis 6, wobei das eutektische Gemisch ausgewählt ist unter:
n-Butylphthalimid/Isopropylphthalimid (60/40 Gew.-%)
n-Butylphthalimid/Isopropylphthalimid (62/38 Gew.-%)
n-Butylphthalimid/Isopropylphthalimid (65/35 Gew.-%)
n-Butylphthalimid/Isopropylphthalimid (70/30 Gew.-%)
n-Butylphthalimid/Isopropylphthalimid (75/25 Gew.-%).

8. Zusammensetzung nach einem der Ansprüche 1 bis 7, wobei das eutektische Gemisch in Konzentrationen von 0,1 bis 50 Gew.-% und noch bevorzugter 1 bis 30 Gew.-%, bezogen auf das Gesamtgewicht der Zusammensetzung, enthalten ist.

9. Zusammensetzung nach einem der Ansprüche 1 bis 8, wobei das 1,3,5-Triazinderivat der folgenden Formel (I) entspricht: 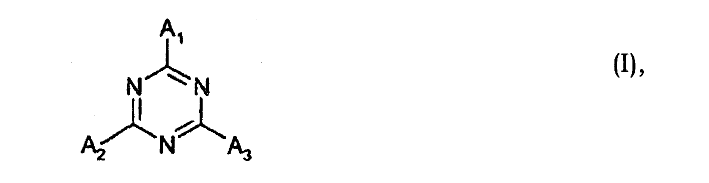 wobei die Gruppen A₁, A₂ und A₃, die gleich oder verschieden sein können, unter den Gruppen der folgenden Formeln (II) bis (IX) ausgewählt sind: 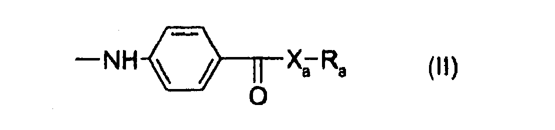 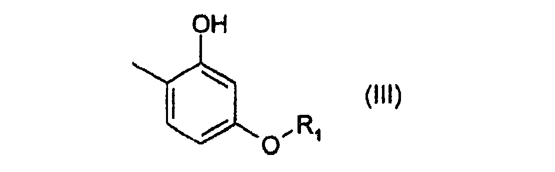 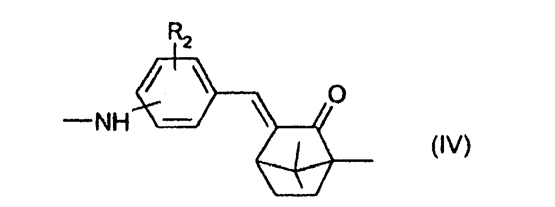 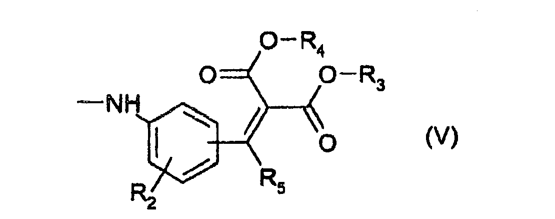 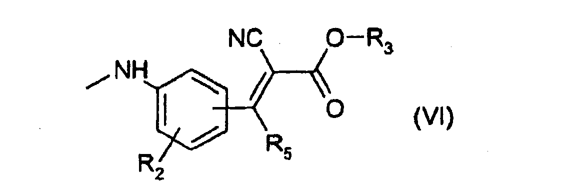 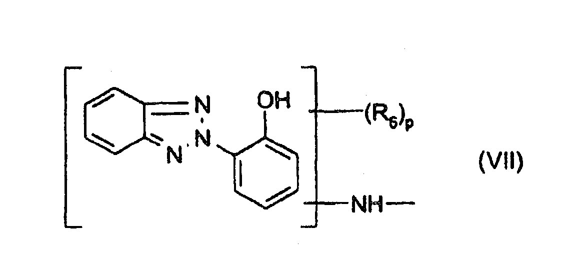 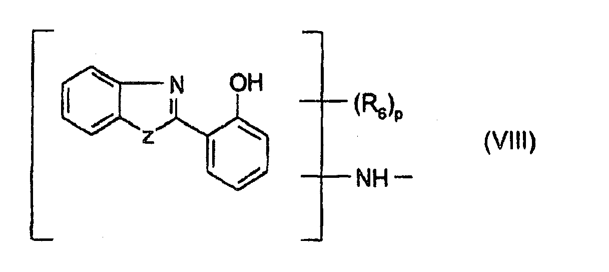 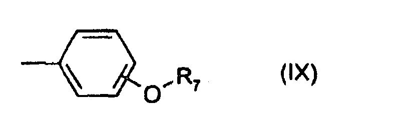 wobei in den Formeln:
- Xₐ (wobei die Gruppen Xₐ gleich oder verschieden sein können) Sauerstoff oder -NH- bedeutet;
- Rₐ (wobei die Gruppen Rₐ gleich oder verschieden sein können) ausgewählt ist unter Wasserstoff; einem Alkalimetall; einer Ammoniumgruppe, die gegebenenfalls mit einer oder mehreren geradkettigen oder verzweigten C₁₋₁₈-Alkylgruppen oder geradkettigen oder verzweigten C₁₋₁₈-Hydroxyalkylgruppen substituiert sein kann; einer linearen oder verzweigten C₁₋₁₈-Alkylgruppe und vorzugsweise C₆₋₁₂-Alkylgruppe; einer C₅₋₁₂-Cycloalkylgruppe, die gegebenenfalls mit einer oder mehreren C₁₋₄-Alkylgruppen substituiert sein kann; einer polyethoxylierten Gruppe mit 1 bis 6 Ethylenoxideinheiten, wobei die endständige OH-Gruppe methyliert ist; einer Gruppe der folgenden Formel (X), (XI) oder (XII): 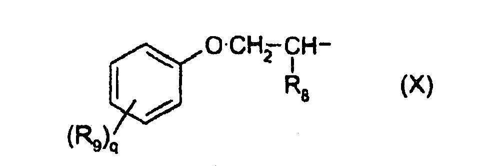 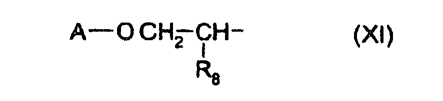 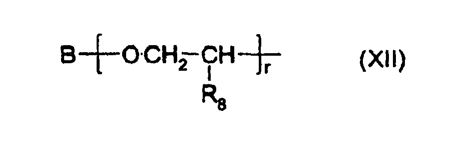 worin bedeuten:
- R₈ Wasserstoff oder Methyl;
- R₉ C₁₋₉-Alkyl;
- q eine ganze Zahl 0; 1; 2; 3;
- r eine ganze Zahl 1; 2; 3; 4; 5; 6; 7; 8; 9; 10;
- A eine C₄₋₈-Alkylgruppe oder eine C₅₋₈-Cycloalkylgruppe;
- B ist ausgewählt unter: einer geradkettigen oder verzweigten C₁₋₈-Alkylgruppe; einer C₅₋₈-Cycloalkylgruppe; einer Arylgruppe, die gegebenenfalls mit einer oder mehreren C₁₋₄-Alkylgruppen substituiert ist;
- R₁ eine C₃₋₁₈-Alkylgruppe; eine C₂₋₁₈-Alkenylgruppe; einen Rest der Formel -CH₂-CH(OH)-CH₂-OT₁, wobei T₁ ein Wasserstoffatom oder eine C₁₋₈-Alkylgruppe bedeutet; einen Rest der folgenden Formel (XIII) bedeutet: 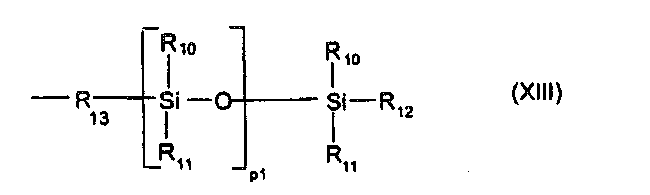 worin bedeuten:
- R₁₃ eine kovalente Bindung; eine lineare oder verzweigte C₁₋₄-Alkylgruppe oder eine Gruppe der Formel -Cₘ₁H₂ₘ₁-O-, worin m₁ eine ganze Zahl 1; 2; 3; 4; bedeutet;
- p₁ eine ganze Zahl gleich 0; 1; 2; 3; 4; 5;
- die Gruppen R₁₀, R₁₁ und R₁₂, die gleich oder verschieden sein können, eine C₁₋₁₈-Alkylgruppe; eine C₁₋₁₈-Alkoxygruppe oder eine Gruppe der Formel: 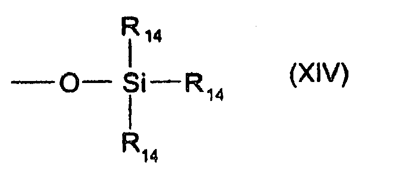
worin R₁₄ eine C₁₋₅-Alkylgruppe ist;
- R₂ ein Wasserstoffatom, eine geradkettige oder verzweigte C₁₋₄-Alkylgruppe oder eine C₁₋₄-Alkoxygruppe bedeutet;
- R₃ und R₄, die gleich oder verschieden sein können, eine geradkettige oder verzweigte C₁₋₂₀-Alkylgruppe bedeuten;
- R₅ ein Wasserstoffatom oder eine Phenylgruppe ist, die gegebenenfalls mit einem Halogen oder einer C₁₋₄-Alkylgruppe oder einer C₁₋₄-Alkoxygruppe substituiert sein kann;
- R₆ eine geradkettige oder verzweigte C₁₋₈-Alkylgruppe oder eine C₁₋₃-Alkoxygruppe, wobei in diesem Fall zwei angrenzende Gruppen R₆ an dem gleich aromatischen Ring gemeinsam eine Alkylidendioxygruppe bilden können, wobei die Alkylidengruppe 1 oder 2 Kohlenstoffatome aufweist, OH, NHCOCH₃ oder NH₂ bedeutet;
- R₇ ein Wasserstoffatom, eine C₁₋₁₀-Alkylgruppe, eine Gruppe der Formel: -(CH₂CHR₅-O)ₙ₁R₈, wobei n₁ eine Zahl von 1 bis 16 bedeutet, oder eine Gruppe der Struktur -CH₂-CH-(OH)-CH₂OT₁ bedeutet, wobei R₈ und T₁ die oben angegebenen Bedeutungen aufweisen;
- Z Sauerstoff, Schwefel, -NH- oder -NR₃- bedeutet, wobei R₃ eine geradkettige oder verzweigte C₁₋₂₀-Alkylgruppe ist;
- p 0, 1, 2 oder 3;
wobei A₁ auch Halogen, eine Gruppe -N(R₃)₂, wobei die beiden Gruppen R₃ gegebenenfalls gemeinsam einen Ring mit 4 oder 5 Kohlenstoffatomen bilden können, oder eine Gruppe -OR₃ bedeuten kann, wobei R₃ die oben angegebenen Bedeutungen aufweist.

10. Zusammensetzung nach Anspruch 9, wobei das 1,3,5-Triazinderivat der Formel (I) entspricht, worin A₁, A₂ und A₃ die Formel (II) aufweisen und alle folgenden Merkmale besitzen:
- eine der Gruppen Xₐ-Rₐ bedeutet die Gruppe -NH-Rₐ, wobei Rₐ ausgewählt ist unter: einer C₅-₁₂-Cycloalkylgruppe, die gegebenenfalls mit einer oder mehreren C₁₋₄-Alkylgruppen substituiert ist; einer Gruppe der Formel (X), (XI) oder (XII) oben, worin bedeuten:
- B eine C₁₋₄-Alkylgruppe;
- R₉ die Methylgruppe;
- die anderen beiden Gruppen Xₐ-Rₐ bedeuten eine Gruppe -O-Rₐ, wobei die Gruppen Rₐ, die gleich oder verschieden sein können, ausgewählt sind unter: Wasserstoff; einem Alkalimetall; einer Ammoniumgruppe, die gegebenenfalls mit einer oder mehreren Alkyl- oder Hydroxyalkylgruppen substituiert ist; einer geradkettigen oder verzweigten C₁₋₁₈-Alkylgruppe, einer C₅₋₁₂-Cycloalkylgruppe, die gegebenenfalls mit einer oder mehreren C₁₋₄-Alkylgruppen substituiert ist; einer Gruppe der Formel (X), (XI) oder (XII) oben, worin bedeuten:
- B eine C₁₋₄-Alkylgruppe;
- R₉ die Methylgruppe.

11. Zusammensetzung nach Anspruch 9, wobei das 1,3,5-Triazinderivat der Formel (I) entspricht, worin A₁, A₂ und A₃ die Formel (II) aufweisen und alle folgenden Merkmale besitzen:
- eine oder zwei Gruppen Xₐ-Rₐ bedeuten die Gruppen -NH-Rₐ, wobei Rₐ ausgewählt ist unter: einer linearen oder verzweigten C₁₋₁₈-Alkylgruppe, einer C₅₋₁₂-Cycloalkylgruppe, die gegebenenfalls mit einer oder mehreren C₁₋₄-Alkylgruppen substituiert ist; eine Gruppe der Formel (X), (XI) oder (XII) oben, worin bedeuten:
- B eine C₁₋₄-Alkylgruppe;
- R₉ die Methylgruppe;
wobei die andere oder die beiden anderen Gruppe(n) Xₐ-Rₐ die Gruppe -O-Rₐ bedeuten, wobei die Gruppen Rₐ, die gleich oder verschieden sein können, ausgewählt sind unter: Wasserstoff; einem Alkalimetall; einer Ammoniumgruppe, die gegebenenfalls mit einer oder mehreren Alkyl- oder Hydroxyalkylgruppen substituiert ist; einer linearen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist; einer Gruppe der Formel (X), (XI) oder (XII) oben, worin bedeuten:
- B eine C₁₋₄-Alkylgruppe;
- R₉ die Methylgruppe.

12. Zusammensetzung nach Anspruch 11, wobei das 1,3,5-Triazinderivat das 2-[(p-*tert*-Butylamido)anilino]-4,6-bis[(p-2'-ethylhexyl-1'-oxycarbonyl)anilino]-1,3,5-triazin der folgenden Formel ist, 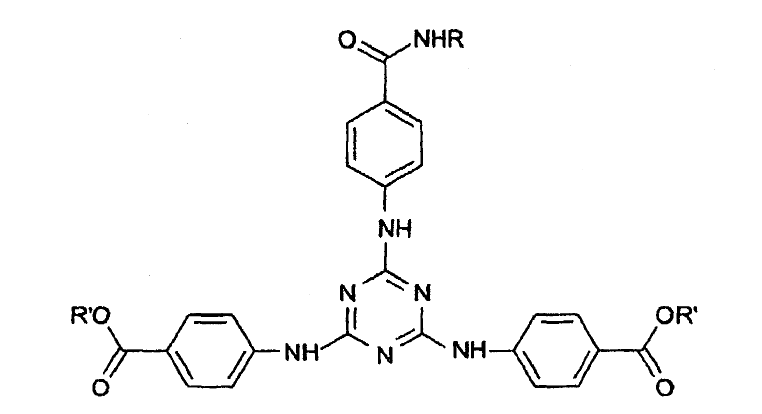 worin R' die 2-Ethylhexylgruppe bedeutet und R die *tert*-Butylgruppe ist.

13. Zusammensetzung nach Anspruch 9, wobei das 1,3,5-Triazinderivat der Formel (I) entspricht, worin A₁, A₂ und A₃ die Formel (II) aufweisen und alle folgenden Merkmale besitzen:
- die Gruppen Xₐ sind gleich und bedeuten Sauerstoff;
- die Gruppen Rₐ, die gleich oder verschieden sein können, bedeuten eine C₆₋₁₂-Alkylgruppe oder eine polyethoxylierte Gruppe mit 1 bis 6 Ethylenoxideinheiten, wobei die endständige OH-Gruppe methyliert ist.

14. Zusammensetzung nach Anspruch 13, wobei das 1,3,5-Triazinderivat das 2,4,6-Tris[p-(2'-ethylhexyl-1'-oxycarbonyl)anilino]-1,3,5-triazin der folgenden Formel ist: 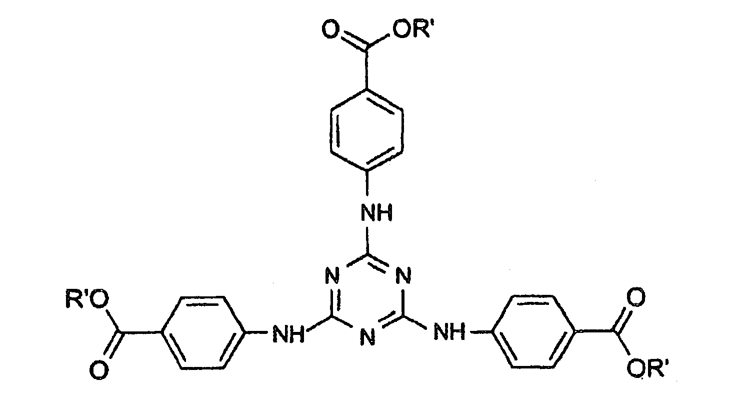 worin R' 2-Ethylhexyl bedeutet.

15. Zusammensetzung nach Anspruch 9, wobei das 1,3,5-Triazinderivat der Formel (I) entspricht, worin A₁ und A₂ die Formel (III) aufweisen und A₃ die Formel (IX) aufweist, mit allen folgenden Merkmalen: die Gruppen R₁, die gleich oder verschieden sein können, bedeuten eine C₃₋₁₈-Alkylgruppe; eine C₂₋₁₈-Alkenylgruppe oder einen Rest der Formel -CH₂-CH(OH)-CH₂-OT₁, wobei T₁ ein Wasserstoffatom oder eine C₁₋₈-Alkylgruppe bedeutet; R₇ bedeutet ein Wasserstoffatom oder eine C₁₋₁₀-Alkylgruppe.

16. Zusammensetzung nach Anspruch 15, wobei das 1,3,5-Triazinderivat das 2,4-Bis{[4-(2-ethylhexyloxy)-2-hydroxy]-phenyl}-6-(4-methoxyphenyl)-1, 3, 5-triazin der folgenden Formel ist: 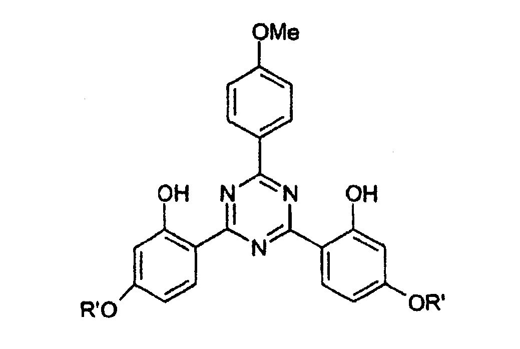 worin R' die 2-Ethylhexylgruppe bedeutet.

17. Zusammensetzung nach Anspruch 9, wobei das 1,3,5-Triazinderivat der Formel (I) entspricht, worin A₁, A₂ und A₃ die Formeln (VII) bis (XI) bedeuten.

18. Zusammensetzung nach Anspruch 17, wobei das 1,3,5-Triazinderivat ausgewählt ist unter:
2,4,6-Tris(diisobutyl-4'-aminobenzalmalonat)-s-triazin,
2,4,6-Tris(bis(2-ethylhexyl)-4'-aminobenzalmalonat)-s-triazin,
2,4,6-Tris(bis(2-ethylhexyl)-4'-aminobenzalmalonat)-6-chlor-s-triazin,
2,4,6-Tris(bis(2-ethylhexyl)-4'-aminobenzalmalonat)-6-(2-ethylhexyl-4'-aminobenzoat)-s-triazin,
2,4,6-Tris(düsobutyl-4'-aminobenzalmalonat)-6-butoxy-s-triazin,
2,4,6-Tris(diisobutyl-4'-aminobenzalmalonat)-6-(2-ethylhexyl-amino)-s-triazin,
2,4-Bis(4'-aminobenzylidencampher)-6-(2-ethylhexylamino)-s-triazin,
2,4-Bis(4'-aminobenzylidencampher)-6-(diisobutyl-4'-aminobenzalmalonat)-s-triazin,
2,4,6-Tris(diethyl-4'-aminobenzalmalonat)-s-triazin,
2,4,6-Tris(diisopropyl-4'-aminobenzalmalonat)-s-triazin,
2,4,6-Tris(dimethyl-4'-aminobenzalmalonat)-s-triazin,
2,4,6-Tris(ethyl-a-cyano-4-aminocinnamat)-s-triazin,
2,4,6-Tris[(3'-benzotriazol-2-yl-2'-hydroxy-5'-methyl)phenyl-amino]-s-triazin,
2,4,6-Tris[(3'-benzotriazol-2-yl-2'-hydroxy-5'-*tert*-octyl)phenyl-amino]-s-triazin.

19. Zusammensetzung nach einem der Ansprüche 1 bis 18, wobei das oder die 1,3,5-Triazinderivat(e) in Konzentrationen von 0,05 bis 15 % und vorzugsweise 0,1 bis 10 Gew.-%, bezogen auf das Gesamtgewicht der Zusammensetzung, enthalten ist (sind).

20. Zusammensetzung nach einem der Ansprüche 1 bis 19, die ferner mindestens ein ergänzendes organisches Lichtschutzmittel und/oder mindestens ein ergänzendes anorganisches Lichtschutzmittel enthält, das wasserlöslich, fettlöslich oder in gewöhnlich verwendeten kosmetischen Lösungsmitteln unlöslich ist.

21. Zusammensetzung nach Anspruch 20, wobei die ergänzenden organischen Lichtschutzmittel unter Anthranilaten; Dibenzoylmethanderivaten; Zimtsäurederivaten; Salicylsäurederivaten; Campherderivaten; Benzophenonderivaten; β, β'-Diphenylacrylat-derivaten; Benzotriazolderivaten; Benzalmalonatderivaten; Benzimidazolderivaten; Imidazolinen; Bis-benzazolylderivaten; Derivaten von p-Aminobenzoesäure (PABA); Methylen-bis(hydroxy-phenylbenzotriazol)-Derivaten; Benzoxazolderivaten; Filterpolymeren und Filtersiliconen; von α-Alkylstyrol abgeleiteten Dimeren; 4,4-Diarylbutadienen und deren Gemischen ausgewählt sind.

22. Zusammensetzung nach Anspruch 21, wobei das ergänzende organische Lichtschutzmittel ausgewählt ist unter:
Homosalate,
Ethylhexyl Salicylate,
Ethylhexyl Methoxycinnamate,
Butylmethoxydibenzoylmethane,
Octocrylene,
Phenylbenzimidazole Sulfonic Acid,
Benzophenone-3,
Benzophenone-4,
Benzophenone-5,
n-Hexyl-2-(4-diethylamino-2-hydroxybenzoyl)-benzoat,
4-Methylbenzylidene Camphor,
Terphthalylidenedicamphor Sulfonic Acid,
Disodium Phenyldibenzimidazole Tetrasulfonate,
Methylen-bis(benzotriazolyl)tetramethylbutylphenol,
Drometrizole Trisiloxane,
Polysilicone-15,
1,1- Dicarboxy-(2,2'-dimethylpropyl)-4,4'-diphenyl- butadien,
2,4-Bis[5-1 (dimethylpropyl)benzoxazol-2-yl-(4-phenyl)-imino]-6-(2-ethylhexyl)-imino-1,3, 5-triazin,
und deren Gemischen.

23. Zusammensetzung nach Anspruch 20, wobei das ergänzende anorganische Lichtschutzmittel unter den beschichteten oder unbeschichteten Metalloxidpigmenten oder Metalloxidnanopigmenten ausgewählt ist.

24. Zusammensetzung nach Anspruch 23, wobei die ergänzenden anorganischen Lichtschutzmittel Nanopigmente von Titanoxid, das amorph oder kristallin in Form von Rutil und/oder Anatas vorliegen kann, oder von Eisenoxid, Zinkoxid, Zirconiumoxid oder Ceroxid sind.

25. Zusammensetzung nach einem der Ansprüche 1 bis 24, **dadurch gekennzeichnet, dass** sie mindestens einen Stoff für die künstliche Bräunung und/oder Braunfärbung der Haut enthält.

26. Zusammensetzung nach einem der Ansprüche 1 bis 25, **dadurch gekennzeichnet, dass** sie ferner mindestens einen Zusatzstoff enthält, der unter den Fettsubstanzen, organischen Lösungsmitteln, ionischen oder nichtionischen, hydrophilen oder lipophilen Verdickungsmitteln, Weichmachern, Feuchthaltemitteln, Trübungsmitteln, Stabilisatoren, Emollientien, Siliconen, Schaumverhütungsmitteln, Duftstoffen, Konservierungsmitteln, anionischen, kationischen, nichtionischen, zwitterionischen oder amphoteren grenzflächenaktiven Stoffen, Wirkstoffen, Füllstoffen, Polymeren, Treibmitteln und Ansäuerungsmitteln oder Alkalisierungsmitteln ausgewählt ist.

27. Zusammensetzung nach einem der Ansprüche 1 bis 26, **dadurch gekennzeichnet, dass** die Kombination eutektisches Gemisch von *n*-Butylphthalimid und Isopropylphthalimid / Öl auf Amidbasis in der Zusammensetzung in einer Menge enthalten ist, die ausreicht, um alleine alle in der Zusammensetzung enthaltende Triazin-Lichtschutzmittel zu lösen.

28. Verwendung der Zusammensetzung nach einem der Ansprüche 1 bis 27 für die Herstellung von kosmetischen Produkten für die Behandlung der Haut, der Lippen, der Nägel, des Haars, der Lider, der Augenbrauen und/oder der Kopfhaut.

29. Verwendung einer Zusammensetzung nach einem der Ansprüche 1 bis 27 für die Herstellung von Produkten für die Pflege der Haut, der Lippen, der Nägel, der Haare und/oder der Kopfhaut.

30. Verwendung einer Zusammensetzung nach einem der Ansprüche 1 bis 27 für die Herstellung von Schminkprodukten.

31. Verwendung einer Kombination aus (i) mindestens einem Öl, das in seiner Struktur mindestens eine Amideinheit aufweist, und (ii) mindestens einem eutektischen Gemisch von *n*-Butylphthalimid und Isopropylphthalimid nach einem der vorhergehenden Ansprüche in einer kosmetischen oder dermatologischen Zusammensetzung, die ein 1, 3, 5-Triazinderivat nach einem der vorhergehenden Ansprüche enthält, um den Lichtschutzfaktor, die kosmetischen Eigenschaften und/oder die Stabilität der Zusammensetzung zu verbessern.

## Revendications

1. Composition cosmétique ou dermatologique photoprotectrice, **caractérisée par le fait qu'**elle comprend :
a) au moins un dérivé de 1,3,5-triazine et
b) au moins une huile présentant dans sa structure au moins un motif amide et
c) au moins un mélange eutectique de n-butylphtalimide et d'isopropylphtalimide.

2. Composition selon la revendication 1, où l'huile présentant dans sa structure au moins un motif amide est choisie parmi les composés de formule (1a) ou (1b) ci-dessous : 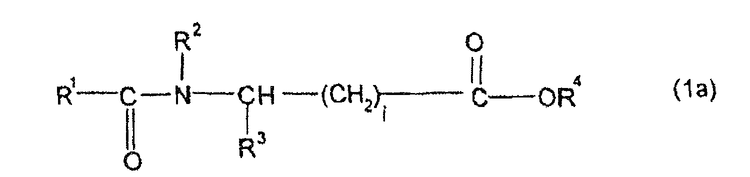 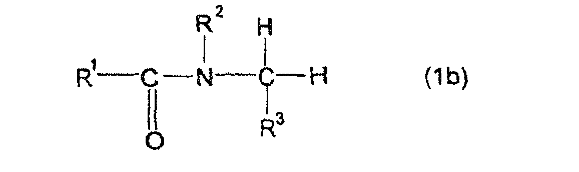 où :
- le radical R¹ représente un radical hydrocarboné monovalent, saturé ou insaturé, aliphatique, cyclo-aliphatique ou cyclique, éventuellement fonctionnalisé, contenant de 1 à 30 atomes de carbone et de préférence de 1 à 22 atomes de carbone, bornes incluses ;
- les radicaux R² R³ et R⁴ qui peuvent être identiques ou différents, représentent des radicaux hydrocarbonés monovalents, saturés ou insaturés, aliphatiques, cycloaliphatiques ou cycliques, éventuellement fonctionnalisés, contenant de 1 à 30 atomes de carbone et de préférence de 1 à 22 atomes de carbone, bornes incluses ;
- i est un nombre entier de 0 à 2.

3. Composition selon la revendication 2, où l'huile présentant dans sa structure au moins un motif amide de formule (1a) ou (1b) est choisie parmi celles où :
R¹ représente un radical alkyle en C₁-C₂₂ linéaire ou ramifié ; un radical alcényle en C₂-C₂₂ linéaire ou ramifié ; un radical aryle ;
R² représente un atome d'hydrogène ou un groupe alkyle en C₁-C₆ linéaire ou ramifié ;
R³ représente un atome d'hydrogène ou un groupe alkyle en C₁-C₆ linéaire ou ramifié ;
R⁴ représente un radical alkyle en C₁ -C₁₀ linéaire ou ramifié, ou un radical alcényle en C₂-C₁₀ linéaire ou ramifié ou un reste stérol.

4. Composition selon la revendication 3, où l'huile présentant dans sa structure au moins un motif amide de formule (1a) ou (1b) est choisie parmi :
le N-acétyl N-butylaminopropionate d'éthyle de formule suivante : 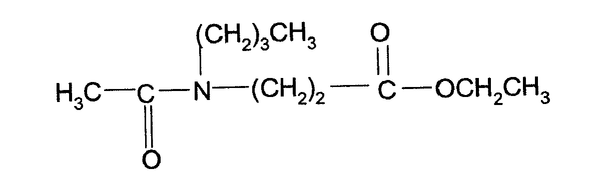
le N-lauroylsarcosinate d'isopropyle de formule : 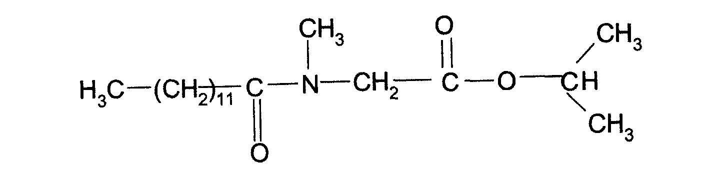
le N,N-diéthyltoluamide de formule :

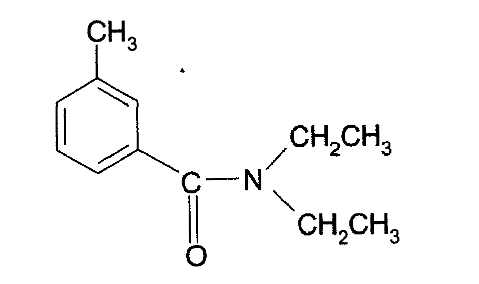

5. Composition selon l'une quelconque des revendications 1 à 4, où l'huile ou les huiles présentant dans leur structure au moins une fonction amide sont présentes à des concentrations allant de préférence de 0,1 à 40 % en poids et plus préférablement de 1 à 20 % en poids par rapport au poids total de la composition.

6. Composition selon l'une quelconque des revendications 1 à 5, où le mélange eutectique est constitué de :
(a) 60 à 75 % en poids de n-butylphtalimide de structure (2) suivante : 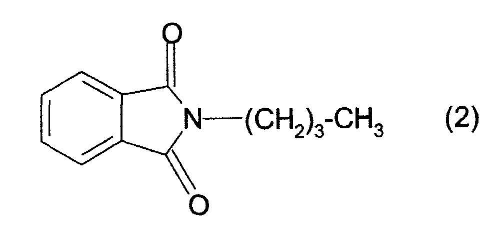
(b) 25 à 40 % en poids d'isopropylphtalimide conforme à l'invention correspondant à la structure (3) suivante :

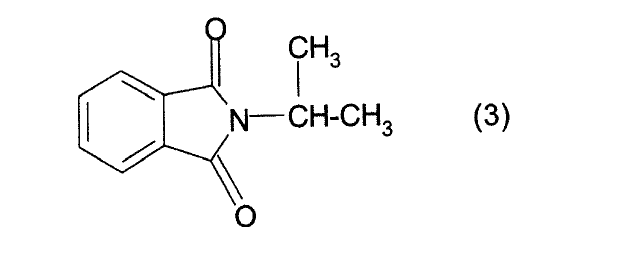

7. Composition selon l'une quelconque des revendications 1 à 6, où le mélange eutectique est choisi parmi :
n-butylphtalimide/isopropylphtalimide (60/40 % en poids)
n-butylphtalimide/isopropylphtalimide (62/38 % en poids)
n-butylphtalimide/isopropylphtalimide (65/35 % en poids)
n-butylphtalimide/isopropylphtalimide (70/30 % en poids)
n-butylphtalimide/isopropylphtalimide (75/25 % en poids)

8. Composition selon l'une quelconque des revendications 1 à 7, où le mélange eutectique est présent à des concentrations allant de 0,1 à 50 % en poids et plus préférablement de 1 à 30 % en poids par rapport au poids total de la composition.

9. Composition selon l'une quelconque des revendications 1 à 8, où le dérivé de 1,3,5-triazine répond à la formule (I) suivante : 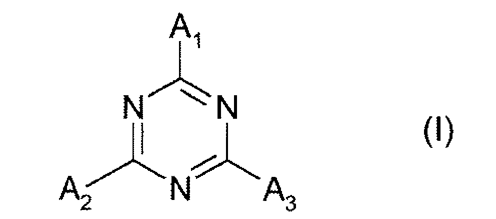 dans laquelle les radicaux A₁, A₂ et A₃, qui peuvent être identiques ou différents, sont choisis parmi les groupes de formules (II) à (IX) ci-dessous : 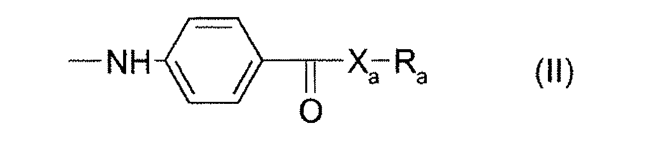 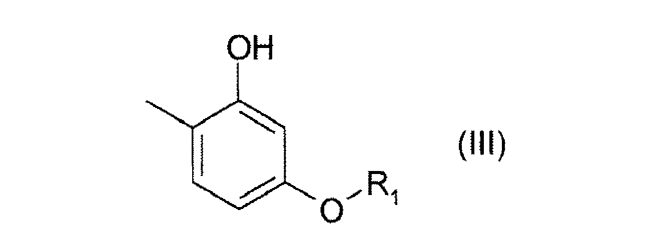 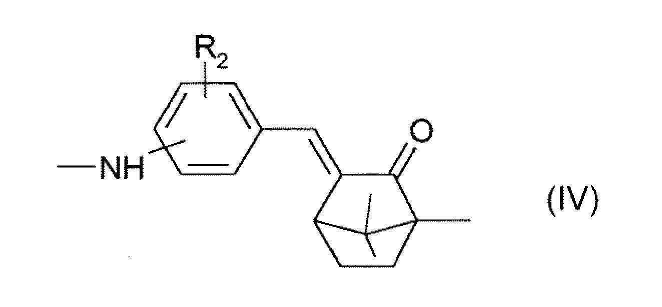 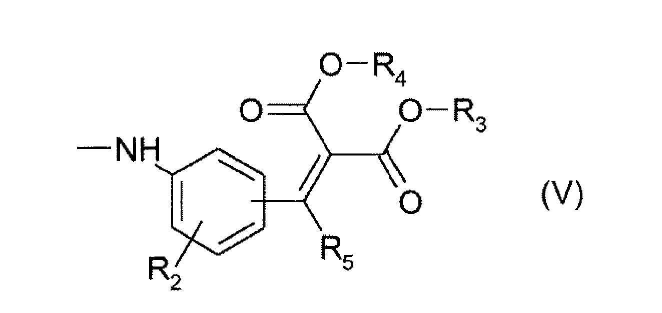 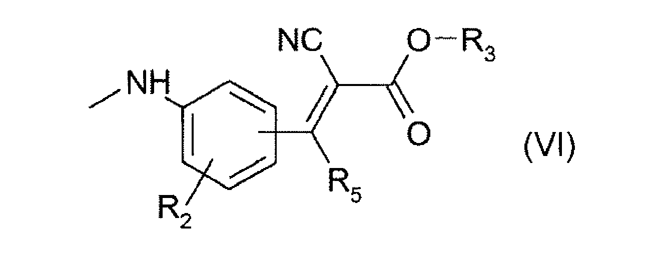 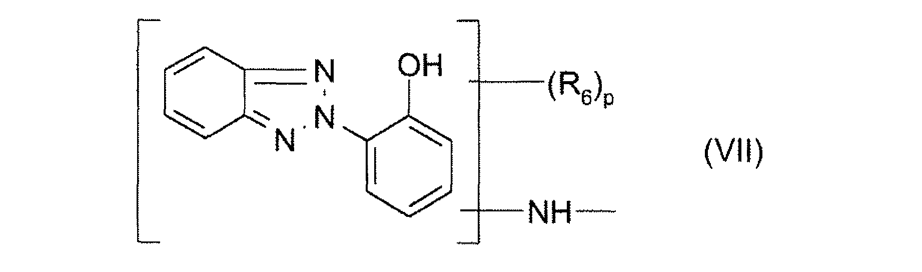 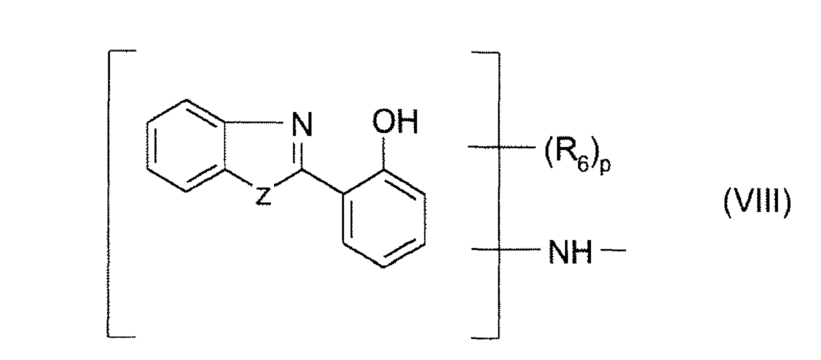 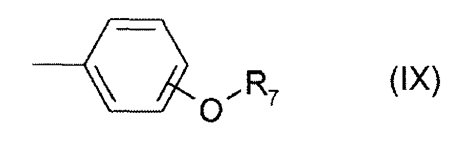 dans lesquelles :
- Xₐ (chacun des groupes Xₐ peut être identique ou différent) représente l'oxygène ou -NH- ;
- Rₐ (chacun des groupes Rₐ peut être identique ou différent) est choisi parmi l'hydrogène ; un métal alcalin ; un radical ammonium éventuellement substitué par un ou plusieurs radicaux alkyle en C₁-C₁₈ linéaires ou ramifiés ou hydroxyalkyle en C₁-C₁₈ linéaires ou ramifiés ; un radical alkyle en C₁-C₁₈ et de préférence en C₆-C₁₂ linéaire ou ramifié ; un radical cycloalkyle en C₅-C₁₂ éventuellement substitué par un ou plusieurs radicaux alkyle en C₁-C₄ ; un radical polyoxyéthyléné comprenant de 1 à 6 unités d'oxyde d'éthylène, dont le groupe OH terminal est méthylé ; un radical de formule (X), (XI) ou (XII) ci-dessous : 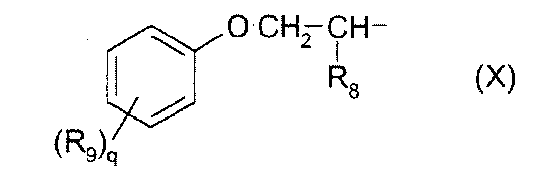 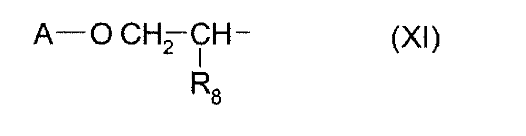 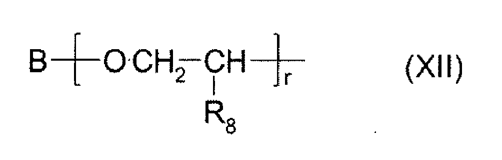
où :
- R₈ est l'hydrogène ou un radical méthyle ;
- R₉ est un radical alkyle en C₁-C₉ ;
- q est un nombre entier égal à 0 ; 1 ; 2 ; 3 ;
- r est un nombre entier égal à 1 ; 2 ; 3 ; 4 ; 5 ; 6 ; 7 ; 8 ; 9 ; 10 ;
- A est un radical alkyle en C₄-C₈ ou un radical cycloalkyle en C₅ - C₈ ;
- B est choisi parmi : un radical alkyle en C₁-C₈ linéaire ou ramifié ; un radical cycloalkyle en C₅-C₈ ; un radical aryle éventuellement substitué par un ou plusieurs radicaux alkyle en C₁-C₄ ;
- R₁ désigne un radical alkyle en C₃-C₁₈ ; un radical alcényle en C₂-C₁₈ ; un reste de formule -CH₂-CH (OH) -CH₂-OT₁ où T₁ est un atome d'hydrogène ou un radical alkyle en C₁-C₈ ; un reste de formule (XIII) suivante : 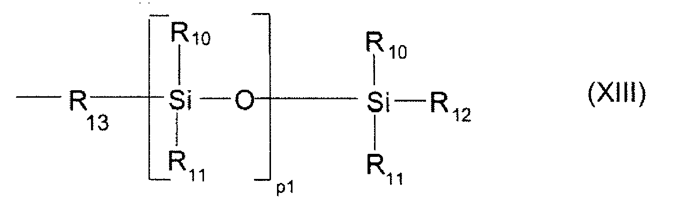
dans laquelle :
- R₁₃ désigne une liaison covalente ; un radical alkyle en C₁-C₄ linéaire ou ramifié ou un radical de formule
- Cₘ₁H₂ₘ₁-O- où m₁ est un nombre entier égal à 1 ; 2 ; 3 ; 4 ;
- p₁ est un nombre entier égal à 0 ; 1 ; 2 ; 3 ; 4 ; 5 ;
- les radicaux R₁₀, R₁₁ et R₁₂, qui peuvent être identiques ou différents, désignent un radical alkyle en C₁-C₁₈ ; un radical alcoxy en C₁-C₁₈ ou un radical de formule : 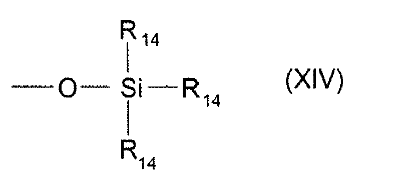
dans laquelle R₁₄ est un radical alkyle en C₁-C₅ ;
- R₂ désigne un atome d'hydrogène, un radical alkyle en C₁-C₄ linéaire ou ramifié ou un radical alcoxy en C₁-C₄ ;
- R₃ et R₄, qui peuvent être identiques ou différents, désignent un radical alkyle en C₁-C₂₀ linéaire ou ramifié ;
- R₅ représente un atome d'hydrogène ou un radical phényle éventuellement substitué par un halogène ou par un radical alkyle en C₁-C₄ ou par un radical alcoxy en C₁-C₄ ;
- R₆ est un radical alkyle en C₁-C₈ linéaire ou ramifié ou un radical alcoxy en C₁-C₃, étant entendu que, dans ce dernier cas, deux radicaux R₆ adjacents d'un même noyau aromatique peuvent former ensemble un groupement alkylidène dioxy dans lequel le groupe alkylidène contient de 1 à 2 atomes de carbone, OH, NHCOCH₃ ou NH₂ ;
- R₇ désigne un atome d'hydrogène, un radical alkyle en C₁-C₁₀, un radical de formule : -(CH₂CHR₅-O)ₙ₁R₈ où n₁ est un nombre de 1 à 16, ou un radical de structure -CH₂-CH-(OH)-CH₂OT₁, R₈ et T₁ ayant la même signification que celle indiquée ci-dessus ;
- Z représente l'oxygène, le soufre, -NH- ou -NR₃-, R₃ représentant un radical alkyle en C₁-C₂₀ linéaire ou ramifié ;
- p est 0, 1, 2 ou 3,
A₁ peut également être un halogène, un radical -N(R₃)₂, les deux radicaux R₃ pouvant former ensemble un cycle de 4 ou 5 atomes de carbone, ou un groupe -OR₃, R₃ ayant la même définition qu'au-dessus.

10. Composition selon la revendication 9, où le dérivé de 1,3,5-triazine répond à la formule (I) dans laquelle les A₁, A₂ et A₃ sont de formule (II) et présentent l'ensemble des caractéristiques suivantes :
- l'un des groupements Xₐ-Rₐ représente un radical
- NH-Rₐ avec Rₐ choisi parmi : un radical cycloalkyle en C₅-C₁₂ éventuellement substitué par un ou plusieurs radicaux alkyle en C₁-C₄ ; un radical de formule (X) , (XI) ou (XII) ci-dessus où :
- B est un radical alkyle en C₁-C₄ ;
- Rg est un radical méthyle ;
- les 2 autres groupements Xₐ-Rₐ représentent un radical -O-Rₐ avec Rₐ, qui peuvent être identiques ou différents, choisis parmi : l'hydrogène ; un métal alcalin ; un radical ammonium éventuellement substitué par un ou plusieurs radicaux alkyle ou hydroxyalkyle ; un radical alkyle en C₁-C₁₈ linéaire ou ramifié ; un radical cycloalkyle en C₅-C₁₂ éventuellement substitué par un ou plusieurs radicaux alkyle en C₁-C₄ ; un radical de formule (X) , (XI) ou (XII) ci-dessus où :
- B est un radical alkyle en C₁-C₄ ;
- Rg est un radical méthyle.

11. Composition selon la revendication 9, où le dérivé de 1,3,5-triazine répond à la formule (I) dans laquelle les A₁, A₂ et A₃ sont de formule (II) et présentent l'ensemble des caractéristiques suivantes :
- un ou deux groupements Xₐ-Rₐ représentent un radical -NH-Rₐ, avec Rₐ choisi parmi : un radical alkyle en C₁-C₁₈ linéaire ou ramifié ; un radical cycloalkyle en C₅-C₁₂ éventuellement substitué par un ou plusieurs radicaux alkyle en C₁-C₄ ; un radical de formule (X), (XI) ou (XII) ci-dessus où :
- B est un radical alkyle en C₁-C₄ ;
- Rg est un radical méthyle ;
l'autre groupement ou les deux autres groupements Xₐ-Rₐ étant un radical -O-Rₐ avec Rₐ, qui peuvent être identiques ou différents, choisis parmi : l'hydrogène ; un métal alcalin ; un radical ammonium éventuellement substitué par un ou plusieurs radicaux alkyle ou hydroxyalkyle ; un radical alkyle en C₁-C₁₈ linéaire ou ramifié ; un radical cycloalkyle en C₅-C₁₂ éventuellement substitué par un ou plusieurs radicaux alkyle en C₁-C₄ ; un radical de formule (X), (XI) ou (XII) ci-dessus où:
- B est un radical alkyle en C₁-C₄ ;
- Rg est un radical méthyle.

12. Composition selon la revendication 11, où le dérivé de 1,3,5-triazine est la 2-[(p-(tertiobutyl-amido)anilino]-4,6-bis-[(p-(2'-éthylhexyl-1'-oxycarbonyl)anilino]-1,3,5-triazine de formule suivante : 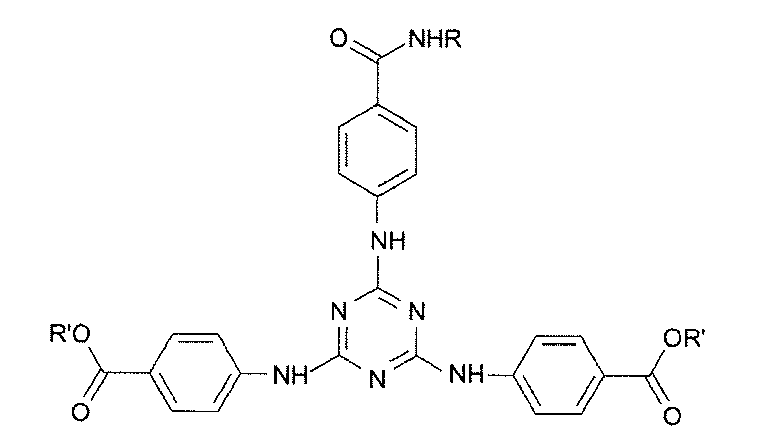 dans laquelle R' désigne un radical 2-éthylhexyle et R désigne un radical tertiobutyle.

13. Composition selon la revendication 9, où le dérivé de 1,3,5-triazine répond à la formule (I) dans laquelle les A₁, A₂ et A₃ sont de formule (II) et présentent l'ensemble des caractéristiques suivantes :
- Xₐ sont identiques et représentent l'oxygène ;
- Rₐ, qui peuvent être identiques ou différents, représentent un radical alkyle en C₆-C₁₂ ou un radical polyoxyéthyléné comprenant de 1 à 6 unités d'oxyde d'éthylène, dont le groupe OH terminal est méthylé.

14. Composition selon la revendication 13, où le dérivé de 1,3,5-triazine est la 2,4,6-tris[p-(2'-éthylhexyl-1'-oxycarbonyl)anilino]-1,3,5-triazine de formule suivante : 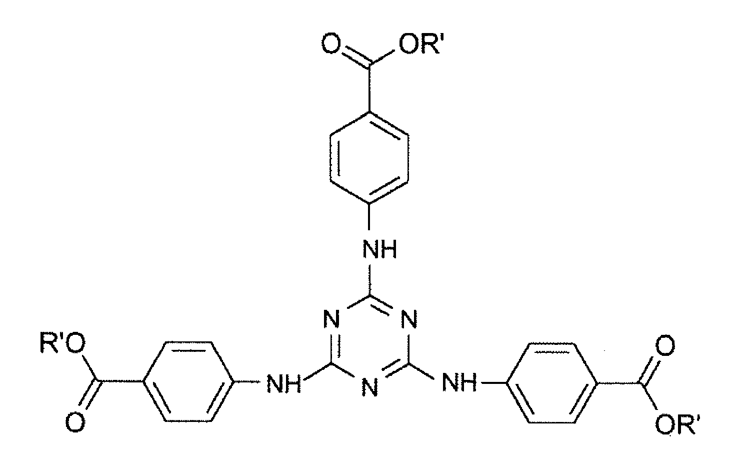 dans laquelle R' désigne un radical 2-éthylhexyle.

15. Composition selon la revendication 9, où le dérivé de 1,3,5-triazine répond à la formule (I) dans laquelle les A₁ et A₂ sont de formule (III) et A₃ est de formule (IX) et présentent l'ensemble des caractéristiques suivantes : R₁, qui peuvent être identiques ou différents, désignent un radical alkyle en C₃-C₁₈ ; un radical alcényle en C₂-C₁₈ ou un reste de formule -CH₂-CH(OH)-CH₂-OT₁ où T₁ est un atome d'hydrogène ou un radical alkyle en C₁-C₈ ; R₇ désigne un atome d'hydrogène ou un radical alkyle en C₁-C₁₀.

16. Composition selon la revendication 15, où le dérivé de 1,3,5-triazine est la 2,4-bis{[4-2-éthylhexyloxy)]-2-hydroxy]-phényl}-6-(4-méthoxyphényl)-1,3,5-triazine de formule suivante: 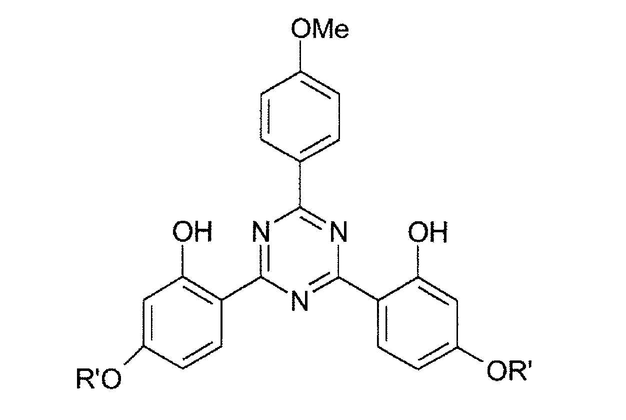 dans laquelle R' désigne un radical 2-éthylhexyle.

17. Composition selon la revendication 9, où le dérivé de 1,3,5-triazine répond à la formule (I) dans laquelle les A₁, A₂ et A₃ sont de formules (VII) à (XI) .

18. Composition selon la revendication 17, où le dérivé de 1,3,5-triazine est choisi parmi :
la 2,4,6-tris-(4'-aminobenzalmalonate de diisobutyle)-s-triazine,
la 2,4,6-tris-(4'-aminobenzalmalonate de di(2-éthylhexyle))-s-triazine,
la 2,4,6-tris-(4'-aminobenzalmalonate de di(2-éthylhexyle))-6-chloro-s-triazine,
la 2,4,6-tris-(4'-aminobenzalmalonate de di(2-éthylhexyle))-6-(4'-aminobenzoate de 2-éthylhexyle)-s-triazine,
la 2,4,6-tris-(4'-aminobenzalmalonate de diisobutyle)-6-butoxy-s-triazine,
la 2,4,6-tris-(4'-aminobenzalmalonate de diisobutyle)-6-(2-éthylhexylamino)-s-triazine,
la 2,4-bis-(4'-aminobenzylidène camphre)-6-(2-éthyl-hexylamino)-s-triazine,
la 2,4-bis-(4'-aminobenzylidène camphre)-6-(4'-aminobenzalmalonate de diisobutyle)-s-triazine,
la 2,4,6-tris-(4'-aminobenzalmalonate de diéthyle)-s-triazine,
la 2,4,6-tris-(4'-aminobenzalmalonate de diisopropyle)-s-triazine,
la 2,4,6-tris-(4'-aminobenzalmalonate de diméthyle)-s-triazine,
la 2,4,6-tris-(α-cyano-4-aminocinnamate d'éthyle)-s-triazine,
la 2,4,6-tris-[(3'-benzotriazol-2-yl-2'-hydroxy-5'-méthyl)phénylamino]-s-triazine,
la 2,4,6-tris-[(3'-benzotriazol-2-yl-2'-hydroxy-5'-tert-octyl)phénylamino]-s-triazine.

19. Composition selon l'une quelconque des revendications 1 à 18, où le ou les dérivés de 1,3,5-triazine sont présents à des concentrations allant de 0,05 à 15 % et de préférence de 0,1 1 à 10 % en poids par rapport au poids total de ladite composition.

20. Composition selon l'une quelconque des revendications 1 à 19, contenant en plus au moins un agent photoprotecteur organique supplémentaire et/ou au moins un agent photoprotecteur minéral supplémentaire, hydrosoluble, liposoluble ou insoluble dans les solvants cosmétiques couramment utilisés.

21. Composition selon la revendication 20, où les agents photoprotecteurs organiques supplémentaires sont choisis parmi les anthranilates ; les dérivés de dibenzoylméthane ; les dérivés cinnamiques ; les dérivés salicyliques ; les dérivés du camphre ; les dérivés de la benzophénone ; les dérivés de β,β'-diphénylacrylate ; les dérivés de benzotriazole ; les dérivés de benzalmalonate ; les dérivés de benzimidazole ; les imidazolines ; les dérivés de bis-benzoazolyle ; les dérivés de l'acide p-aminobenzoïque (PABA) ; les dérivés de méthylène bis-(hydroxyphényl benzotriazole) ; les dérivés de benzoxazole ; les polymères filtres et silicones filtres ; les dimères dérivés d'α-alkylstyrène ; les 4,4-diarylbutadiènes et leurs mélanges.

22. Composition selon la revendication 21, où les filtres organiques supplémentaires sont choisis parmi :
- l'homosalate,
- le salicylate d'éthylhexyle,
- le méthoxycinnamate d'éthylhexyle,
- le méthoxydibenzoylméthane de butyle,
- l'octocrylène,
- l'acide phénylbenzimidazole sulfonique,
- la benzophénone-3,
- la benzophénone-4,
- la benzophénone-5,
- le 2-(4-diéthylamino-2-hydroxybenzoyl)-benzoate de n-hexyle,
- le 4-méthylbenzylidène camphre,
- l'acide téréphtalylidène dicamphre sulfonique,
- le phényl dibenzimidazole tétrasulfonate de disodium,
- le méthylène bis-benzotriazolyl tétraméthylbutylphénol,
- le drométrizole trisiloxane,
- le polysilicone-15,
- le 1,1-dicarboxy(2,2'-diméthylpropyl)-4,4-diphénylbutadiène,
- la 2,4-bis-[5-1(diméthylpropyl)benzoxazol-2-yl-(4-phényl)imino]-6-(2-éthylhexyl)imino-1,3,5-triazine, et leurs mélanges.

23. Composition selon la revendication 20, où les agents photoprotecteurs minéraux supplémentaires sont choisis parmi des pigments ou des nanopigments d'oxydes métalliques, enrobés ou non.

24. Composition selon la revendication 23, où les filtres minéraux supplémentaires sont des nanopigments d'oxyde de titane, amorphe ou cristallisé, sous forme rutile et/ou anatase, de fer, de zinc, de zirconium ou de cérium.

25. Composition selon l'une quelconque des revendications 1 à 24, **caractérisée par le fait qu'**elle comprend en outre au moins un agent de bronzage et/ou de brunissage artificiel de la peau.

26. Composition selon l'une quelconque des revendications 1 à 25, **caractérisée par le fait qu'**elle comprend en outre au moins un adjuvant choisi parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non ioniques, zwitterioniques ou amphotères, des actifs, les charges, les polymères, les propulseurs et les agents alcalinisants ou acidifiants.

27. Composition selon l'une quelconque des revendications 1 à 26, **caractérisée par le fait que** l'association mélange eutectique de n-butylphtalimide et d'isopropylphtalimide/huile amidée est présente dans la composition dans une quantité suffisante pour solubiliser à elle seule la quantité totale de filtre triazine présent dans la composition.

28. Utilisation d'une composition telle que définie dans l'une quelconque des revendications 1 à 27, pour la fabrication de produits pour le traitement cosmétique de la peau, des lèvres, des ongles, des cheveux, des cils, des sourcils et/ou du cuir chevelu.

29. Utilisation d'une composition telle que définie dans l'une quelconque des revendications 1 à 27, pour la fabrication de produits de soin de la peau, des lèvres, des ongles, des cheveux et/ou du cuir chevelu.

30. Utilisation d'une composition telle que définie dans l'une quelconque des revendications 1 à 27, pour la fabrication de produits de maquillage.

31. Utilisation d'une association de (i) au moins une huile présentant dans sa structure au moins un motif amide et (ii) au moins un mélange eutectique de n-butylphtalimide et d'isopropylphtalimide tel que défini dans l'une quelconque des revendications précédentes, dans une composition cosmétique ou dermatologique contenant un dérivé de 1,3,5-triazine tel que défini dans les revendications précédentes, en vue d'améliorer le facteur de protection solaire, les propriétés cosmétiques et/ou la stabilité de cette composition.
